# EUROPEAN PATENT APPLICATION

(11) **EP 4 541 785 A1**
(43) Date of publication of application: **23.04.2025**
(21) Application number: 23823145.0
(22) Date of filing: 13.06.2023
(51) Int. Cl.: C07C 229/16, C07C 227/06, A61K 9/127, A61K 48/00

(54) **CATIONIC LIPID COMPOUND, AND PREPARATION METHOD THEREFOR AND USE THEREOF**

(30) Priority: 14.06.2022 CN 202210667897
(71) Applicant: Highfield Biopharmaceuticals Corporation, HangZhou, Zhejiang 310051 (CN)
(72) Inventor: WU, Kangzeng, Hangzhou, Zhejiang 310051 (CN); XU, Fengwei, Hangzhou, Zhejiang 310051 (CN); DAI, Yongchao, Hangzhou, Zhejiang 310051 (CN); LIU, Yu, Hangzhou, Zhejiang 310051 (CN); XU, Yuhong, Hangzhou, Zhejiang 310051 (CN)
(74) Representative: CMS Cameron McKenna Nabarro Olswang LLP
(86) International application number: PCT/CN2023/099945
(87) International publication number: WO 2023/241577

(57) **Abstract**

The present application relates to the field of drug carriers, and specifically discloses a cationic lipid compound, and a preparation method therefor and a use thereof. The cationic lipid compound comprises a compound represented by formula I; in the formula I, n is equal to 1, 2, 3 or 4; R1 and R2 are each independently selected from secondary long-chain alkyl esters represented by formula II; in the formula II, n₁=2, 4 or 6, n₂=5, 7, 9 or 11, and n₃=5, 7, 9 or 11. The cationic lipid compound is combined with phospholipids, cholesterol and polyethylene glycol lipids, such that an LNP carrier may be prepared, and then nucleic acid molecules are wrapped to prepare a corresponding drug. The raw materials for the production of the cationic lipid compound in the present application are easy to obtain, the synthesis steps are simple, the constructed LNP carrier has a good drug delivery effect, the corresponding drug can efficiently enter a target cell or activate an immune response of an organism, and thus the cationic lipid compound has practical application value.

## Description

### Cross-Reference to Related Applications

The present disclosure claims priority to Chinese Patent Application No. 2022106678978 filed on 14 June 2022. The content disclosed in the present disclosure is incorporated herein by reference in its entirety.

### Technical Field

The present disclosure relates to the technical field of drug carriers, and more specifically relates to a cationic lipid compound, a preparation method therefor, and use thereof.

### Background

As a new technology born from the combination of modern medicine and molecular biology, gene therapy refers to introduction of a target gene into a body of a patient to correct or compensate for a disease caused by a defective and abnormal gene, so that the disease can be treated. As a new means for disease treatment, gene therapy has achieved some successful uses, and the achieved scientific breakthroughs will continue to promote the development of gene therapy toward mainstream medical care. Common gene drugs include plasmid DNA (pDNA), antisense oligonucleotide (antisense ODN), small interfering RNA (siRNA), small hairpin RNA (shRNA), and messenger RNA (mRNA). mRNA is a type of single-stranded ribonucleic acid containing particular genetic information, and can transfer the genetic information it carries to ribosomes in cells, where it serves as a template to guide organisms to synthesize particular target proteins. Compared with conventional protein drugs that can hardly run across cell membranes, the mechanism of action of mRNA enables it to deliver transmembrane or intracellular proteins to the organisms, so that it is possible to treat or prevent more diseases. Due to the above-mentioned advantages, mRNA also becomes a trend in future drug development.

The key to gene therapy likes in delivering a gene drug to target cells in vivo, so that the gene drug can take effect. However, an exogenous gene, when directly introduced in vivo, will be degraded by nuclease in vivo, and will be degraded into small molecule nucleotides prior to entering the target cells, thus losing therapeutic effects. Therefore, the key to realizing gene therapy lies in constructing an efficient and safe gene delivery system.

A gene carrier needs to transport genes through a plurality of complex processes: reaching the target cells through blood circulation, cellular uptake, endosomal escape, intracellular movement, and release of gene materials from the carrier. The main obstacles are extracellular obstacles in a complex blood environment and intracellular barriers to lysosomal enzyme degradation. Therefore, how to find a good gene carrier so that the target gene can function when reaching a target spot is an urgent problem for gene carrier researchers.

At present, gene delivery vector systems are mainly classified into two categories: one is a viral vector system, and the other is a non-viral vector system. Viral vector is a natural vector resource. Viral genome is characterized by a simple structure, high transfection efficiency, and strong target cell specificity, but suffers from poor guidance, low carrying capacity, immunogenicity, and potential tumorigenicity, making it difficult to achieve clinical use requirements. Therefore, in recent years, the non-viral vector system characterized by diversity, non-immunogenicity, easy control and easy production has attracted much attention, and has been applied in many therapeutic fields.

A common non-viral vector system is mainly lipid carrier. Lipid carrier usually contains positive charges of a cationic lipid, and combines with a negatively charged gene drug through electrostatic interaction, thereby concentrating and encapsulating the gene materials into particles of smaller particle sizes, thus forming lipid nanoparticles (LNPs). Compared to other types of liposomes, LNPs show incomparable advantages in the preparation of gene delivery vectors and in cell transfection. A smaller particle size of the complex reduces the chance of being recognized, phagocytized, and cleared by macrophages in vivo, and improves in vivo bioavailability of drugs. Moreover, for tumor tissues, the smaller particle size of the complex makes it easier to penetrate from vascular endothelial cell spaces into tumor parenchyma through penetration and retention effects, thereby increasing drug aggregation in the tumor tissues. In terms of transfection, since cell surface is slightly negatively charged, positively charged liposomes are easier to be adsorbed to the cell surface, and enter cells through mechanisms such as endocytosis, thereby greatly increasing transfection ability of the liposomes.

At present, characterized by simple structure, easy operation, and high biosecurity, LNP has become a most widely used non-viral vector. However, most preparation processes of LNP are complex, and it is difficult to expand production. Therefore, how to provide a non-viral vector system with a simple preparation method and good therapeutic effects has become an urgent problem.

### Summary of the Invention

In order to improve the drug delivery effects of gene carriers and simplify the synthesis steps thereof, the present disclosure provides a cationic lipid compound, a preparation method therefor, and use thereof. This type of cationic lipid compound is positively charged, and is easier to enter cells, thereby better delivering drugs, and producing better therapeutic effects, is characterized by simple synthesis and easy preparation, and has practical use value.

In a first aspect, the present disclosure provides a cationic lipid compound, adopting the following technical solutions:
a cationic lipid compound, comprising a compound represented by formula I; 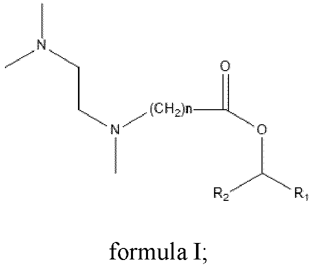
wherein, in the formula I, n=1, 2, 3, or 4; and
R₁ and R₂ are each independently selected from a secondary long-chain alkyl ester represented by formula II; 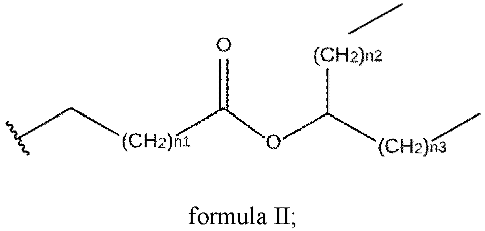
wherein, in the formula II, n₁=2, 4, or 6; n₂=5, 7, 9, or 11; n₃=5, 7, 9, or 11; and a wavy line represents a connection point between the formula II and other moieties of the formula I.

In the present disclosure, an amphipathic derivative obtained by combining 3-((2-(dimethylamino)ethyl)(methyl)amino)propionic acid and a secondary long-chain alkyl ester serves as a cationic lipid component in a carrier system for a gene drug, and is positively charged, thereby better delivering the gene drug, and achieving better gene therapy effects. This type of cationic lipid compound can achieve large-scale production due to simple synthesis steps and readily available raw materials.

In the present disclosure, the cationic lipid compound comprises 3 ester groups in total. Spatial positions of the 3 ester groups directly affect functioning of the cationic lipid compound, and the number of carbon atoms on the alkyl chain will affect spatial structure of the ester groups. As experimentally verified, when n=1, 2, 3, or 4, n₁=2, 4, or 6, n₂=5, 7, 9, or 11, and n₃=5, 7, 9, or 11, the cationic compound has best loading and delivery effects on the gene drug, thereby achieving best therapeutic effects.

As a preferred technical solution, the cationic lipid compound according to the present disclosure comprises a compound represented by formula III, formula IV, formula V, formula VI, formula VII, formula VIII, or formula IX.

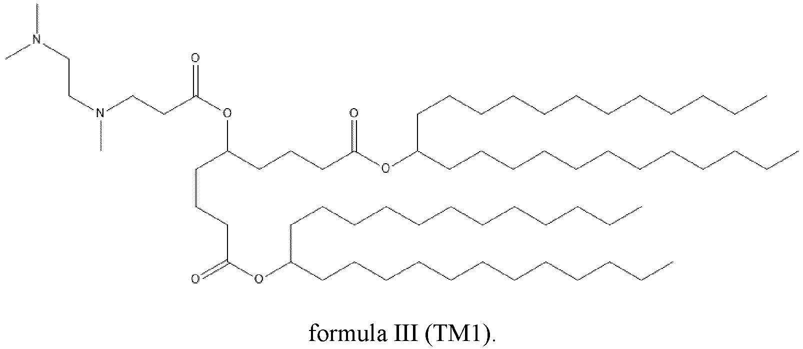

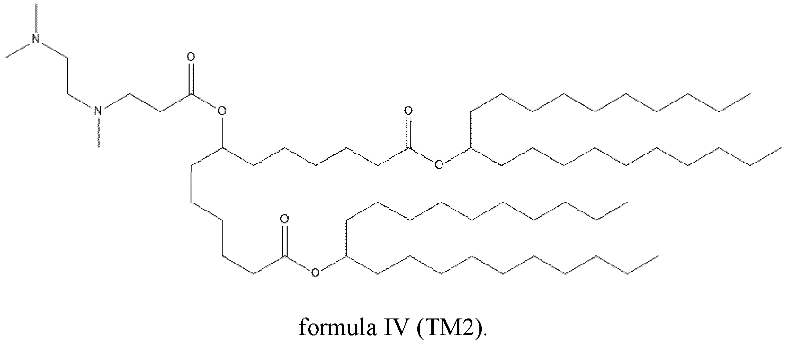

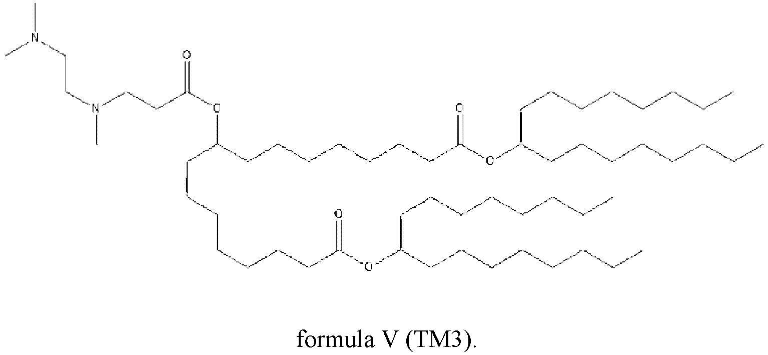

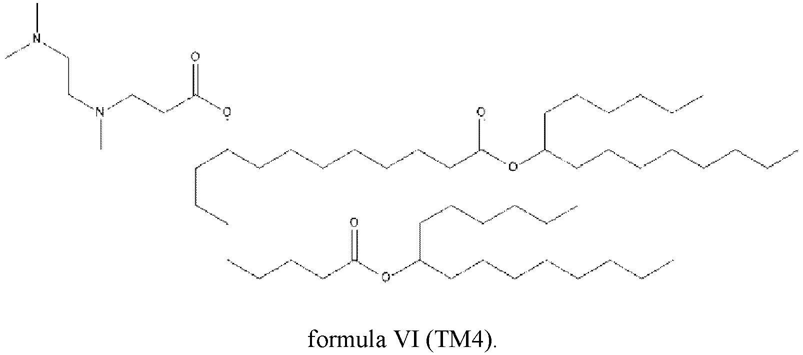

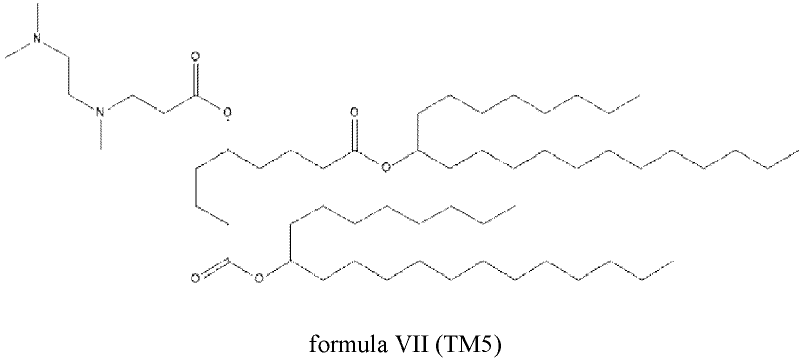

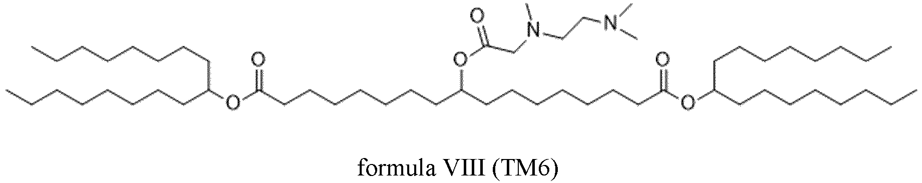

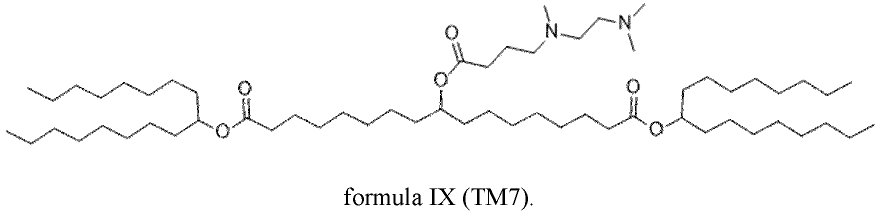

In a second aspect, the present disclosure provides a method for preparing a cationic lipid compound, adopting the following technical solutions:
A method for preparing a cationic lipid compound, comprising steps of:
sequentially performing an alkylation reaction and a reduction reaction with a bromoester compound as a raw material,
followed by a condensation reaction with an acyl chloride compound, sequentially performing a two-step substitution reaction and a hydrolysis reaction, and finally performing an esterification reaction with a monohydric secondary alcohol compound to obtain the cationic lipid compound.

As a preferred technical solution, the method for preparing a cationic lipid compound includes the following steps:
(1) generating an intermediate product 2 through an alkylation reaction of a bromoester compound with TosMIC (p-toluenesulfonylmethyl isocyanide) and NaH;
(2) generating an intermediate product 3 through a reaction of the intermediate product 2 under an acidic condition;
(3) generating an intermediate product 4 through a reduction reaction of the intermediate product 3 with NaBH₄;
(4) generating an intermediate product 5 through a condensation reaction of the intermediate product 4 with an acyl chloride compound;
(5) generating an intermediate product 6 through a substitution reaction of the intermediate product 5 with NaI;
(6) generating an intermediate product 7 through a substitution reaction of the intermediate product 6 with N,N,N'-trimethylethylenediamine;
(7) generating an intermediate product 8 through a hydrolysis reaction of the intermediate product 7 with LiOH; and
(8) obtaining the cationic lipid compound through an esterification reaction of the intermediate product 8 with a monohydric secondary alcohol compound.

In the present disclosure, the raw material in the step (1) is a bromoester compound, and may be specifically selected in an actual production process based on the structure of the to-be-synthesized specific cationic lipid compound.

Preferably, the bromoester compound includes ethyl 8-bromooctanoate, ethyl 6-bromocaproate, or ethyl 4-bromobutyrate.

Preferably, in the step (1), TBAI (tetrabutylammonium iodide) is used as a catalyst for the reaction.

Preferably, in the step (1), the reaction solvent includes a DMSO (dimethyl sulfoxide) solution.

Preferably, in the step (1), the reaction is carried out at room temperature.

Preferably, in the step (2), the reaction solvent includes a mixed solution of DCM (dichloromethane) and concentrated sulfuric acid at a volume ratio of DCM: concentrated sulfuric acid=(3.5-8): 1.

In the present disclosure, the concentrated sulfuric acid provides an acidic environment for the reaction in the step (2).

Preferably, in the step (2), the reaction is carried out at room temperature.

In the present disclosure, the intermediate product 3 is a symmetric ketone compound.

Preferably, in the step (3), the reaction solvent includes a mixed solution of THF (tetrahydrofuran) and ethanol at a volume ratio of THF: ethanol=(2-4):1.

Preferably, in the step (3), the reaction is carried out at room temperature.

In the present disclosure, the intermediate product 4 is an alcohol compound.

In the present disclosure, the acyl chloride compound in the step (4) is a collective term for a class of compounds containing different numbers of carbon atoms and a terminal unsubstituted with halogen, and may be specifically selected in an actual production process based on the structure of the to-be-synthesized specific cationic lipid compound.

Preferably, the acyl chloride compound includes 3-chloropropionyl chloride, 2-chloroacetyl chloride, 4-chlorobutyryl chloride, or 5-chlorovaleryl chloride.

Preferably, in the step (4), the reaction solvent includes a pyridine-containing DCM solution, wherein the concentration of pyridine is 73-134 mM.

Preferably, in the step (4), the reaction is carried out at room temperature.

Preferably, in the step (5), the reaction solvent includes an acetone solution.

Preferably, in the step (5), the reaction is carried out at 68-72°C, and the reaction temperature may be, for example, 68°C, 70°C, 72°C, 68-70°C, 68-72°C, 70-72°C, or the like.

Preferably, in the step (6), the reaction solvent includes a DCM solution.

Preferably, in the step (6), the reaction is carried out at room temperature.

Preferably, in the step (7), the reaction solvent includes a mixed solution of ethanol and water at a volume ratio of ethanol: water=1:(1.5-3).

Preferably, in the step (7), the reaction is carried out at room temperature.

Preferably, in the step (8), DMAP (4-dimethylaminopyridine) and DCC (dicyclohexylcarbodiimide) are used as catalysts for the reaction.

Preferably, in the step (8), the reaction solvent includes a DCM solution.

Preferably, in the step (8), the reaction is carried out at room temperature.

In the present disclosure, in the step (8), the monohydric secondary alcohol compound is a monohydric alcohol containing different numbers of carbon atoms with a hydroxyl not at position 1, and may be specifically selected in an actual production process based on the structure of the to-be-synthesized specific cationic lipid compound.

Preferably, the monohydric secondary alcohol compound includes 13-pentacosanol, 11-heneicosanol, 9-heptadecanol, 7-pentadecanol, or 9-heneicosanol.

In a third aspect, the present disclosure provides a LNP carrier, adopting the following technical solutions:
a LNP carrier, comprising any one or a combination of at least two of the cationic lipid compound according to the first aspect.

In the present disclosure, the cationic lipid compound in the LNP carrier may be a single cationic lipid compound, or may be a combination of a plurality of cationic lipid compounds.

Preferably, by molar fraction, a molar fraction of the cationic lipid compound in the LNP carrier is 15%-70%, for example, may be 15%, 30%, 45%, 60%, 70%, 15%-30%, 15%-45%, 15%-60%, 15%-70%, 30%-45%, 30%-60%, 30%-70%, 45%-60%, 45%-70%, 60%-70%, or the like. This molar fraction is a molar ratio of a lipid material in the LNP carrier, excluding contained drugs (such as a nucleic acid drug).

In the present disclosure, when the cationic lipid compound in the LNP carrier is a single cationic lipid compound, its molar fraction in the LNP carrier is 15%-70% (for example, 15%, 30%, 45%, 60%, 70%, 15%-30%, 15%-45%, 15%-60%, 15%-70%, 30%-45%, 30%-60%, 30%-70%, 45%-60%, 45%-70%, 60%-70%, or the like); and when the cationic lipid compound in the LNP carrier is a combination of a plurality of cationic lipid compounds, a total molar fraction of the combination of the plurality of cationic lipid compounds in the LNP carrier is 15%-70% (for example, 15%, 30%, 45%, 60%, 70%, 15%-30%, 15%-45%, 15%-60%, 15%-70%, 30%-45%, 30%-60%, 30%-70%, 45%-60%, 45%-70%, 60%-70%, or the like). In some aspects, the molar fraction described above is a molar ratio of a lipid material in the LNP carrier, excluding contained drugs (such as a nucleic acid drug).

In some aspects, the LNP carrier further comprises 5%-30% (for example, 5%-20% or 8%-30%) of phospholipid and 15%-65% of cholesterol by molar fraction. This molar fraction is a molar ratio of a lipid material in the LNP carrier, excluding contained drugs (such as a nucleic acid drug).

In some aspects, the LNP carrier further comprises 5%-30% (for example, 5%-20% or 8%-30%) of phospholipid, 15%-65% of cholesterol, and 1.5%-3% (for example, 1%-3% or 0.5%-2%) of polyethylene glycol lipid by molar fraction. This molar fraction is a molar ratio of a lipid material in the LNP carrier, excluding contained drugs (such as a nucleic acid drug).

Preferably, a molar fraction of the phospholipid in the LNP carrier is 5%-40%, for example, may be 8%, 10%, 15%, 20%, 25%, 30%, 5%-10%, 8%-10%, 5%-15%, 8%-15%, 5%-20%, 8%-20%, 5%-25%, 8%-25%, 5%-30%, 8%-30%, 10%-15%, 10%-20%, 10%-25%, 10%-30%, 15%-20%, 15%-25%, 15%-30%, 20%-25%, 20%-30%, 25%-30%, or the like. This molar fraction is a molar ratio of a lipid material in the LNP carrier, excluding contained drugs (such as a nucleic acid drug).

Preferably, a molar fraction of the cholesterol in the LNP carrier is 10%-65%, for example, may be 10%, 15%, 30%, 45%, 65%, 15%-30%, 15%-45%, 15%-65%, 30%-45%, 30%-65%, 45%-65%, or the like. This molar fraction is a molar ratio of a lipid material in the LNP carrier, excluding contained drugs (such as a nucleic acid drug).

In some aspects, by molar fraction, the LNP carrier further comprises polyethylene glycol lipid, wherein a molar fraction of the polyethylene glycol lipid in the LNP carrier is 0.5%-3%, for example, may be 1.5%, 2%, 2.5%, 3%, 0.5%-2%, 1%-2%, 1.5%-2%, 0.5%-2.5%, 1%-2%, 1.5%-2.5%, 0.5%-3%, 1%-2%, 1.5%-3%, 2%-2.5%, 1%-3%, 2%-3%, 2.5%-3%, or the like. This molar fraction is a molar ratio of a lipid material in the LNP, excluding contained drugs (such as a nucleic acid drug).

In some aspects of the present disclosure, by molar fraction, the LNP carrier comprises 15%-70% (for example, 15%, 30%, 45%, 60%, 70%, 30%-70%, 15%-30%, 15%-45%, 15%-60%, 15%-70%, 30%-45%, 30%-60%, 30%-70%, 45%-60%, 45%-70%, 60%-70%, or the like) of a cationic lipid compound, 5%-30% (for example, 8%, 10%, 15%, 20%, 25%, 30%, 5%-10%, 8%-10%, 5%-15%, 8%-15%, 5%-20%, 8%-20%, 5%-25%, 8%-25%, 5%-30%, 8%-30%, 10%-15%, 10%-20%, 10%-25%, 10%-30%, 15%-20%, 15%-25%, 15%-30%, 20%-25%, 20%-30%, 25%-30%, or the like) of phospholipid, and 10%-65% (for example, 15%, 30%, 45%, 65%, 15%-30%, 15%-45%, 15%-65%, 30%-45%, 30%-65%, 45%-65%, or the like) of cholesterol. In some aspects, by molar fraction, the LNP carrier comprises 15%-70% (for example, 15%, 30%, 45%, 60%, 70%, 30%-70%, 15%-30%, 15%-45%, 15%-60%, 15%-70%, 30%-45%, 30%-60%, 30%-70%, 45%-60%, 45%-70%, 60%-70%, or the like) of a cationic lipid compound, 5%-30% (for example, 8%, 10%, 15%, 20%, 25%, 30%, 5%-10%, 8%-10%, 5%-15%, 8%-15%, 5%-20%, 8%-20%, 5%-25%, 8%-25%, 5%-30%, 8%-30%, 10%-15%, 10%-20%, 10%-25%, 10%-30%, 15%-20%, 15%-25%, 15%-30%, 20%-25%, 20%-30%, 25%-30%, or the like) of phospholipid, 10%-65% (for example, 15%, 30%, 45%, 65%, 15%-30%, 15%-45%, 15%-65%, 30%-45%, 30%-65%, 45%-65%, or the like) of cholesterol, and 0.5%-2% (for example, 1.5%, 2%, 2.5%, 3%, 0.5%-2%, 1%-2%, 1.5%-2%, 0.5%-2.5%, 1%-2%, 1.5%-2.5%, 0.5%-3%, 1%-2%, 1.5%-3%, 2%-2.5%, 2%-3%, 2.5%-3%, or the like) of polyethylene glycol lipid. This molar fraction is a molar ratio of a lipid material in the LNP, excluding contained drugs (such as a nucleic acid drug).

In the present disclosure, ingredients of the LNP carrier and respective addition amounts thereof are optimized, to produce a technical effect of synergistic gain, and achieve higher loading amount of the nucleic acid drug, more controllable particle size distribution of the LNP, and stronger transfection effects of target cells, thereby improving the drug efficacy, reducing the toxic and side effects, and achieving better therapeutic effects.

In a fourth aspect, the present disclosure provides a drug, adopting the following technical solutions:
a drug, comprising the LNP carrier according to the third aspect.

Preferably, the drug further comprises a nucleic acid drug. In some aspects, the drug is a gene drug. In some aspects, the gene drug includes, but is not limited to, plasmid DNA (pDNA), antisense oligonucleotide (antisense ODN), small interfering RNA (siRNA), small hairpin RNA (shRNA), and messenger RNA (mRNA). In some aspects, the drug is a mRNA. In some aspects, the drug is a mRNA encoding SARS-CoV-2 spike glycoprotein (S protein).

Preferably, in the drug, a molar ratio of nitrogen content of the cationic lipid compound in the LNP carrier to phosphorus content in the nucleic acid drug (i.e., N/P molar ratio) is (2-15):1, for example, may be 2:1, 3:1, 4:1, 5:1, 6:1, (2-3):1, (2-4):1, (2-5):1, (2-6):1, (3-4):1, (3-5):1, (3-6):1, (4-5):1, (4-6):1, (5-6):1, or the like.

In some aspects, in the drug, the nucleic acid drug (for example, a mass fraction (drug loading amount) of the mRNA encoding SARS-CoV-2 spike glycoprotein) in the drug-loaded LNP is 1%-10%, for example, may be 1%-7%, 1%-6%, 2%-6%, 2%-5%, 3%-5%, or the like.

In the present disclosure, a ratio of the cationic lipid compound to the nucleic acid drug directly affects an encapsulation rate of the drug and subsequent drug release effects. In some other aspects of the present disclosure, when the molar ratio of the nitrogen content of the cationic lipid compound in the LNP carrier to the phosphorus content in the nucleic acid drug is (2-15):1, the drug-loaded structure has better stability and best intracellular release efficiency. In some aspects of the present disclosure, as experimentally verified, when the molar ratio of the nitrogen content of the cationic lipid compound in the LNP carrier to the phosphorus content of the nucleic acid drug is (2-6):1, the drug has a highest encapsulation rate and best therapeutic effects, thereby improving the utilization rate of raw materials, and saving the production costs. In some aspects of the present disclosure, when a mass fraction (drug loading amount) of the nucleic acid drug in the drug-loaded LNP is 3%-10%, the drug has a better encapsulation rate, thereby improving the utilization rate of raw materials, and saving the production costs.

In the present disclosure, a particle size and a particle size distribution of the drug affect the drug loading capacity and subsequent practical use. In some aspects of the present disclosure, an average particle size of the drug is 30 nm-300 nm, for example, may be 30 nm-270 nm, 30 nm-250 nm, 30 nm-200 nm, 50 nm-200 nm, 50 nm-150 nm, 60 nm-120 nm, or the like. In some aspects of the present disclosure, a polydispersity (PDI) of the drug is less than 0.15, for example, may be less than 0.13, less than 0.12, less than 0.11, less than 0.1, less than 0.09, or the like. In some aspects of the present disclosure, a polydispersity (PDI) of the drug is 0.02-0.25, for example, may be 0.02-0.20, 0.05-0.20, 0.05-0.25, 0.05-0.1, 0.05-0.25, or the like. In some aspects of the present disclosure, a PDI of the drug is less than 0.1.

In some aspects of the present disclosure, the drug comprises a LNP carrier and a mRNA (such as a mRNA encoding SARS-CoV-2 spike glycoprotein), wherein a drug loading amount of the mRNA is 2-6% (wt.), wherein the LNP carrier comprises 60 mol%-70 mol% of lipid compound TM3, 7 mol%-12 mol% of phospholipid (such as HSPC), 20 mol%-25 mol% of cholesterol, and 1 mol%-2 mol% of DSPE-PEG2000. In some aspects, an average particle size of the above drug is between 150 nm and 200 nm. In some aspects, a particle size distribution PDI of the above drug is less than 0.1. In some aspects, an encapsulation rate of the above drug is greater than 85%. In some aspects, an N/P value of the above drug is 5.5-6.5.

In some aspects of the present disclosure, the drug comprises a LNP carrier and a nucleic acid drug, wherein a mass ratio of the nucleic acid drug to the LNP carrier is 5%-7%, wherein the LNP carrier comprises 60 mol%-70 mol% of lipid compound TM3, 10 mol%-15 mol% of phospholipid (such as HSPC), 15 mol%-25 mol% of cholesterol, and 1 mol%-2 mol% of DSPE-PEG2000. In some aspects, an average particle size of the above drug is between 50 nm and 100 nm. In some aspects, a PDI of the above drug is less than 0.1 or less than 0.05. In some aspects, an encapsulation rate of the above drug is greater than 80% or greater than 90%.

In some aspects of the present disclosure, the drug comprises a LNP carrier and a nucleic acid drug, wherein a mass ratio of the nucleic acid drug to the LNP carrier is 4%-5%, wherein the LNP carrier comprises 60 mol%-70 mol% of lipid compound TM3, 5 mol%-8 mol% of phospholipid (such as HSPC), 25 mol%-35 mol% of cholesterol, and 1 mol%-2 mol% of DSPE-PEG2000. In some aspects, an average particle size of the above drug is between 100 nm and 150 nm. In some aspects, a PDI of the above drug is less than 0.1. In some aspects, an encapsulation rate of the above drug is greater than 80% or greater than 90%.

In some aspects of the present disclosure, the drug comprises a LNP carrier and a nucleic acid drug, wherein a mass ratio of the nucleic acid drug to the LNP carrier is 4%-5%, wherein the LNP carrier comprises 60 mol%-70 mol% of lipid compound TM3, 10 mol%-15 mol% of phospholipid (such as HSPC), 15 mol%-25 mol% of cholesterol, and 2 mol%-3 mol% of DSPE-PEG2000. In some aspects, an average particle size of the above drug is between 100 nm and 150 nm. In some aspects, an average particle size of the above drug is between 100 nm and 150 nm. In some aspects, a PDI of the above drug is less than 0.1. In some aspects, an encapsulation rate of the above drug is greater than 80% or greater than 90%.

In some aspects of the present disclosure, the drug comprises a LNP carrier and a nucleic acid drug, wherein a mass ratio of the nucleic acid drug to the LNP carrier is 2%-3%, wherein the LNP carrier comprises 25 mol%-35 mol% of lipid compound TM3, 5 mol%-12 mol% of phospholipid (such as HSPC), 50 mol%-60 mol% of cholesterol, and 1 mol%-2 mol% of DSPE-PEG2000. In some aspects, an average particle size of the above drug is between 50 nm and 100 nm. In some aspects, a PDI of the above drug is less than 0.1. In some aspects, an encapsulation rate of the above drug is greater than 80% or greater than 90%.

In some aspects of the present disclosure, the drug comprises a LNP carrier and a nucleic acid drug, wherein a mass ratio of the nucleic acid drug to the LNP carrier is 5%-6%, wherein the LNP carrier comprises 35 mol%-45 mol% of lipid compound TM3, 12 mol%-18 mol% of phospholipid (such as HSPC), 40 mol%-50 mol% of cholesterol, and 0.5 mol%-1 mol% of DSPE-PEG2000. In some aspects, an average particle size of the above drug is between 100 nm and 150 nm. In some aspects, a PDI of the above drug is less than 0.1. In some aspects, an encapsulation rate of the above drug is greater than 80% or greater than 90%.

In some aspects of the present disclosure, the drug comprises a LNP carrier and a nucleic acid drug (such as a mRNA encoding SARS-CoV-2 spike glycoprotein), wherein the LNP carrier comprises 60 mol%-70 mol% of lipid compound TM6, 10 mol%-15 mol% of phospholipid (such as HSPC), 15 mol%-25 mol% of cholesterol, and 2 mol%-3 mol% of DSPE-PEG2000. In some aspects, an average particle size of the above drug is between 100 nm and 150 nm. In some aspects, a particle size distribution PDI of the above drug is less than 0.1. In some aspects, an encapsulation rate of the above drug is greater than 90%. In some aspects, an N/P value of the above drug is 5.5-6.2.

In some aspects of the present disclosure, the drug comprises a LNP carrier and a nucleic acid drug (such as a mRNA encoding SARS-CoV-2 spike glycoprotein), wherein an N/P value is 5.5-6.2, wherein the LNP carrier comprises 25 mol%-35 mol% of lipid compound TM6, 5 mol%-15 mol% of phospholipid (such as HSPC), 55 mol%-65 mol% of cholesterol, and 1 mol%-2 mol% of DSPE-PEG2000. In some aspects, an average particle size of the above drug is between 150 nm and 200 nm. In some aspects, a particle size distribution PDI of the above drug is less than 0.1. In some aspects, an encapsulation rate of the above drug is greater than 85%.

In some aspects of the present disclosure, the drug comprises a LNP carrier and a nucleic acid drug (such as a mRNA encoding SARS-CoV-2 spike glycoprotein), wherein an N/P value is 3.8-4.2, wherein the LNP carrier comprises 45 mol%-55 mol% of lipid compound TM6, 5 mol%-15 mol% of phospholipid (such as HSPC), 35 mol%-45 mol% of cholesterol, and 1 mol%-2 mol% of DSPE-PEG2000. In some aspects, an average particle size of the above drug is between 50 nm and 100 nm. In some aspects, a particle size distribution PDI of the above drug is less than 0.1. In some aspects, an encapsulation rate of the above drug is greater than 85%.

In some aspects of the present disclosure, the drug comprises a LNP carrier and a nucleic acid drug (such as a mRNA encoding SARS-CoV-2 spike glycoprotein), wherein an N/P value is 5.5-6.2, wherein the LNP carrier comprises 60 mol%-70 mol% of lipid compound TM7, 10 mol%-15 mol% of phospholipid (such as HSPC), 15 mol%-25 mol% of cholesterol, and 2 mol%-3 mol% of DSPE-PEG2000. In some aspects, an average particle size of the above drug is between 100 nm and 150 nm. In some aspects, a particle size distribution PDI of the above drug is less than 0.15. In some aspects, an encapsulation rate of the above drug is greater than 70%.

In some aspects of the present disclosure, the drug comprises a LNP carrier and a nucleic acid drug (such as a mRNA encoding SARS-CoV-2 spike glycoprotein), wherein an N/P value is 5.5-6.2, wherein the LNP carrier comprises 25 mol%-35 mol% of lipid compound TM7, 5 mol%-15 mol% of phospholipid (such as HSPC), 55 mol%-65 mol% of cholesterol, and 1 mol%-2 mol% of DSPE-PEG2000. In some aspects, an average particle size of the above drug is between 50 nm and 1100 nm. In some aspects, a particle size distribution PDI of the above drug is less than 0.1. In some aspects, an encapsulation rate of the above drug is greater than 95%.

In some aspects of the present disclosure, the drug comprises a LNP carrier and a nucleic acid drug (such as a mRNA encoding SARS-CoV-2 spike glycoprotein), wherein an N/P value is 3.8-4.2, wherein the LNP carrier comprises 45 mol%-55 mol% of lipid compound TM7, 5 mol%-15 mol% of phospholipid (such as HSPC), 35 mol%-45 mol% of cholesterol, and 1 mol%-2 mol% of DSPE-PEG2000. In some aspects, an average particle size of the above drug is between 100 nm and 150 nm. In some aspects, a particle size distribution PDI of the above drug is less than 0.1. In some aspects, an encapsulation rate of the above drug is greater than 90%.

In some aspects, the drug in the present disclosure can be used to treat or prevent some diseases, such as COVID infections, viral and bacterial infections, tumors, metabolic diseases, autoimmune diseases, cardiovascular diseases, and genetic diseases.

In a fifth aspect, the present disclosure provides a method for preparing the above drug, adopting the following technical solutions:
a method for preparing a drug, comprising steps of:
weighing and dissolving ingredients other than the cationic lipid compound, to obtain a stock solution;
adding the cationic lipid compound to the stock solution, and fully mixing the mixture to obtain an alcoholic lipid phase;
diluting a nucleic acid drug to obtain an aqueous phase of nucleic acid;
mixing the alcoholic lipid phase and the aqueous phase of nucleic acid to obtain a drug intermediate, and dialyzing the drug intermediate to obtain the drug.

Preferably, when the phospholipid, cholesterol, and polyethylene glycol lipid are dissolved, an employed solvent includes ethanol (such as anhydrous ethanol).

Preferably, when the nucleic acid drug is diluted, an acidic buffer saline solution is used for dilution, for example, an acidic buffer saline solution at pH=4.0 is used for dilution.

In the present disclosure, dialysis is performed using a dialysis apparatus to remove the solvent. In some aspects, after the dialysis step, the preparation method of the present disclosure further includes dispersing a LNP in a biocompatible injection buffer.

In a sixth aspect, the present disclosure provides a vaccine, adopting the following technical solutions:
a vaccine, comprising the LNP carrier according to the third aspect.

In a seventh aspect, the present disclosure provides use of the cationic lipid compound according to the first aspect in the preparation of a LNP carrier, a drug, or a vaccine, or use of the LNP carrier according to the third aspect in the preparation of a drug and/or a vaccine.

To sum up, the present disclosure has the following beneficial effects:
1. In the present disclosure, an amphipathic derivative obtained by combining 3-((2-(dimethylamino)ethyl)(methyl)amino)propionic acid and a secondary long-chain alkyl ester serves as a cationic lipid component in a carrier system for a gene drug, and is positively charged, thereby making it easier to be adsorbed to cell surface and enter cells, more efficiently delivering drugs, and achieving, in some aspects, more efficient gene transfection and better therapeutic effects. The raw materials for production are readily available, the preparation method is simple with a high synthesis efficiency, thereby creating conditions for large-scale production; and
2. The LNP carrier prepared using the above cationic lipid compound has good drug loading and delivery efficiency, can reach an encapsulation rate of more than 88% in some aspects, and can, after being made into a drug with a nucleic acid molecule, efficiently express related proteins in vivo, thereby functioning as a therapeutic drug to treat a disease or as a vaccine to activate immunity, and having broad application prospects. In some aspects, the LNP carrier prepared using the above cationic lipid compound can accurately and efficiently enter cells and express related proteins to achieve therapeutic effects.

### Description of Drawings

FIG. 1 is a schematic diagram of a synthetic route of a cationic lipid compound TM3 in the present disclosure.
FIG. 2 is a picture of a hydrogen spectrum detection result of an intermediate product 2 in Preparation Example 1 of the present disclosure.
FIG. 3 is a picture of a hydrogen spectrum detection result of an intermediate product 3 in Preparation Example 1 of the present disclosure.
FIG. 4 is a picture of a hydrogen spectrum detection result of an intermediate product 4 in Preparation Example 1 of the present disclosure.
FIG. 5 is a picture of a hydrogen spectrum detection result of an intermediate product 5 in Preparation Example 1 of the present disclosure.
FIG. 6 is a picture of a hydrogen spectrum detection result of an intermediate product 7 in Preparation Example 1 of the present disclosure.
FIG. 7 is a picture of a hydrogen spectrum detection result of a cationic lipid compound TM3 in Preparation Example 1 of the present disclosure.
FIG. 8 is a picture of a detection result of a protein expression amount of a drug TP1 in 293T cells in Example 1 of the present disclosure.
FIG. 9 is a picture of a detection result of an anti-S protein antibody titer in mouse serum in Example 2 of the present disclosure.
FIG. 10 is a structural diagram of HG-06 LNP under a cryo-electron microscope in Example 3 of the present disclosure.
FIG. 11 is a structural diagram of HG-23 LNP under a cryo-electron microscope in Example 3 of the present disclosure.
FIG. 12 is an image of fluorescently labeled HG-23 LNP entering cells and aggregating in endosomes in Example 3 of the present disclosure.
FIG. 13 is a picture of a hydrogen spectrum detection result of a cationic lipid compound TM6 in an example of the present disclosure.
FIG. 14 is a comparison of cell transfection results of TM6 and TM7 LNP in a plurality of recipes.
FIG. 15 is a comparison of antibody levels induced after immunization of mice with TM6 and TM7 LNP in a plurality of recipes.

### Detailed Description

The present disclosure provides a cationic lipid compound, comprising a compound represented by formula I; 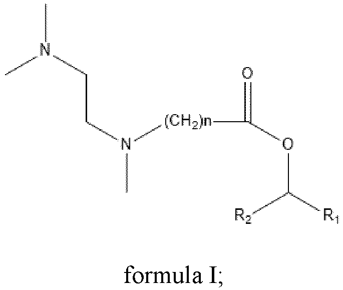
wherein, in the formula I, n=1, 2, 3, or 4; and
R₁ and R₂ are each independently selected from a secondary long-chain alkyl ester represented by formula II; 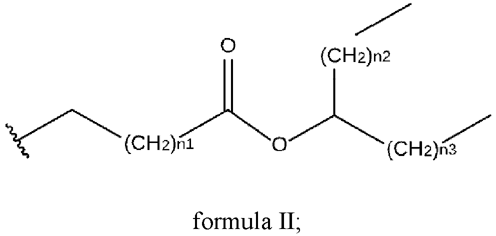
wherein, in the formula II, n₁=2, 4, or 6; n₂=5, 7, 9, or 11; n₃=5, 7, 9, or 11; and a wavy line represents a connection point between the formula II and other moieties of the formula I.

In some aspects of the present disclosure, n=1, 2, or 3.

In some aspects of the present disclosure, n=2.

In some aspects of the present disclosure, the n1 in the R₁ has a same value as the n1 in the R₂.

Specifically, the cationic lipid compound comprises TM1 represented by formula III, TM2 represented by formula IV, TM3 represented by formula V, TM4 represented by formula VI, or TM5 represented by formula VII.

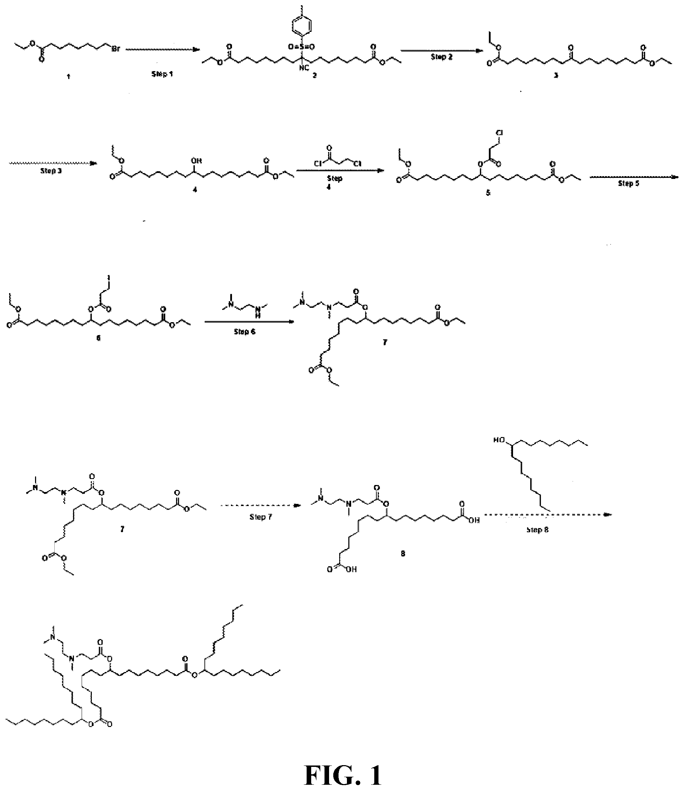

Specifically, in the present disclosure, a method for preparing the cationic lipid compound is illustrated with TM3 as an example. The schematic diagram of its synthetic route is as shown in FIG. 1, and the specific process is as follows:
(1) generating an intermediate product 2 through an alkylation reaction of ethyl 8-bromooctanoate with TosMIC and NaH in a DMSO solution with TBAI as a catalyst at room temperature:
   18-22 mmol of ethyl 8-bromooctanoate is dissolved in 55-65 mL of anhydrous DMSO, the resulting solution is stirred at 8-15°C for 5-10 min, 8-12 mmol of TosMIC is added, the mixture is stirred for 5-10 min, 23-28 mmol of NaH is added batchwise, and finally 1.8-2.2 mmol of TBAI is added. The mixture is slowly warmed to room temperature, and stirred for 1-3 h.
   The reaction extent is monitored by TLC. When the reaction is complete, the system is cooled in an ice water bath, and 145-155 mL of ice water is added to quench the reaction. Then, the mixture is extracted 3 times with 90-110 mL of DCM each time. All organic phases are collected, washed with 90-110 mL of water, then washed twice with 145-155 mL of saturated sodium bicarbonate each time, dried, concentrated, and then purified through a silica gel column to provide the intermediate product 2.
(2) generating an intermediate product 3 through a reaction of the intermediate product 2 in an acidic mixed solution of DCM and concentrated sulfuric acid (volume ratio: (3.5-8):1) at room temperature:
   3.9-4.2 g of the intermediate product 2 is weighed, dissolved in 45-55 mL of a DCM solution, and stirred for 3-7 min. 7-12 mL of concentrated sulfuric acid is added, and the mixture is stirred at room temperature for 2-5 h.
   The reaction extent is monitored by TLC. After the reaction is complete, 45-55 mL of water is added to the system. The mixture is fully mixed, and then left to stand for layering. The aqueous layer is extracted with 45-55 mL of DCM. All organic phases are combined, eluted with 45-55 mL of saturated sodium bicarbonate, dried, concentrated, and then purified through a silica gel column to provide the intermediate product 3.
(3) generating an intermediate product 4 through a reduction reaction of the intermediate product 3 with NaBH₄ in a mixed solution of THF and ethanol (volume ratio: (2-4):1) at room temperature:
   3-5 mmol of the intermediate product 3 is weighed, and dissolved in 45-55 mL of a mixed solution of THF and ethanol (volume ratio: (2-4):1). The mixture is stirred at 0-4°C for 3-7 min. 3-5 mmol of NaBH₄ is slowly added batchwise, and the mixture is kept at room temperature for reaction for 3-5 h.
   The reaction extent is monitored by TLC. After the reaction is complete, 90-110 mL of ice water is added to the system to quench the reaction. The mixture is extracted 3 times with 90-110 mL of DCM each time. All organic phases are combined, eluted with 45-55 mL of saturated sodium bicarbonate, dried, concentrated, and then purified through a silica gel column to provide the intermediate product 4.
(4) generating an intermediate product 5 through a condensation reaction of the intermediate product 4 with 3-chloropropionyl chloride in a pyridine-containing DCM solution (pyridine concentration: 73-134 mM) at room temperature:
   4-6 mmol of the intermediate product 4 is weighed, and dissolved in 45-55 mL of the DCM solution. Then, 4-6 mmol of pyridine is added, the mixture is stirred at 0-4°C for 3-7 min, 7-9 mmol of 3-chloropropionyl chloride is slowly added, and the mixture is kept at room temperature for reaction for 0.5-2 h.
   The reaction extent is monitored by TLC. After the reaction is complete, 90-110 mL of ice water is added to the system to quench the reaction. The mixture is washed with 90-110 mL of water, then washed twice with 140-160 mL of a saturated sodium bicarbonate solution each time, dried, concentrated, and then purified through a silica gel column to provide the intermediate product 5.
(5) generating an intermediate product 6 through a substitution reaction of the intermediate product 5 with NaI in an acetone solution at 68-72°C:
   3-5 mmol of the intermediate product 5 is weighed, and dissolved in 45-55 mL of an acetone solution. 3-6 mmol of NaI is slowly added. The mixture is heated to a temperature of 68-72°C for reaction for 12-18 h.
   The reaction extent is monitored by TLC. After the reaction is complete, the organic phase is eluted with 45-55 mL of saturated sodium thiosulfate, dried, concentrated, and then purified through a silica gel column to provide the intermediate product 6.
(6) generating an intermediate product 7 through a substitution reaction of the intermediate product 6 with N,N,N'-trimethylethylenediamine in a DCM solution at room temperature:
   3-5 mmol of the intermediate product 6 is weighed, and dissolved in 45-55 mL of a DCM solution. 5-7 mmol of N,N,N'-trimethylethylenediamine is added, and the mixture is stirred at room temperature for reaction for 1-3 h.
   The reaction extent is monitored by TLC. After the reaction is complete, the organic phase is eluted with 45-55 mL of saturated sodium bicarbonate, dried, concentrated, and then purified through a silica gel column to provide the intermediate product 7.
(7) generating an intermediate product 8 through a hydrolysis reaction of the intermediate product 7 with LiOH in a mixed solution of ethanol and water (volume ratio: 1:(1.5-3)) at room temperature:
   4-6 mmol of the intermediate product 7 and 18-22 mmol of LiOH are weighed and dissolved in 90-110 mL of a mixed solution of ethanol and water (volume ratio: 1:(1.5-3)). The mixture is stirred at room temperature for 4-7 h.
   The reaction extent is monitored by TLC. After the reaction is complete, and after ethanol is spin-dried, the mixture is acidized with 1.5-2.5 M of hydrochloric acid to a pH of about 2, and extracted 3 times with 90-110 mL of DCM each time. All organic phases are combined, eluted with 45-55 mL of saturated sodium bicarbonate, dried, concentrated, and then purified through a silica gel column to provide the intermediate product 8.
(8) generating the cationic lipid compound TM3 through an esterification reaction of the intermediate product 8 with 9-heptadecanol in a DCM solution under the action of DMAP and DCC at room temperature:
   4-6 mmol of the intermediate product 8, 14-16 mmol of DMAP, and 16-20 mmol of 9-heptadecanol are weighed and dissolved in 55-65 mL of DCM. The mixture is stirred for 3-7 min. 13-17 mmol of DCC is added, and the mixture is stirred at room temperature overnight.

The reaction extent is monitored by TLC. After the reaction is complete, the mixture is washed 3 times with 55-65 mL of water each time, then washed twice with 45-55 mL of saturated sodium bicarbonate each time, dried, concentrated, and then purified through a silica gel column to provide the cationic lipid compound TM3.

The present disclosure further provides a LNP carrier. By molar fraction, the LNP carrier comprises 15%-70% of a cationic lipid compound, 8%-30% of phospholipid, 15%-65% of cholesterol, and 1.5%-3% of polyethylene glycol lipid.

The present disclosure further provides a LNP carrier. By molar fraction, the LNP carrier comprises 15%-70% of a cationic lipid compound, 8%-40% of phospholipid, and 10%-65% of cholesterol.

The present disclosure further provides a drug, comprising a LNP carrier and a nucleic acid drug, wherein a drug loading amount of the nucleic acid drug in the drug is between 1.0-10.0%. In some aspects, a molar ratio of nitrogen content of the cationic lipid compound in the LNP carrier to phosphorus content in the nucleic acid drug is from 2:1 to 15:1, from 2:1 to 10:1, from 2:1 to 6:1, or from 4:1 to 6:1.

In some aspects, the drug may be prepared by:
accurately weighing a lipid based on a recipe, and dissolving the lipid in ethanol while stirring on a magnetic stirrer, adding a calculated amount of a citrate buffer at pH=4 to a mRNA stock solution, fully mixing the mixture,
sucking the above ethanolic lipid solution and an aqueous solution of mRNA-citrate buffer with a syringe, starting a LNP mixing pump to collect a solution in a stable mixing stage, adding the above mixed mRNA-LNP intermediate into a dialysis bag, adding a dialysis buffer pre-cooled at 4°C, transferring the dialysis bag into a 2-8°C refrigerator, stirring at 200 rpm for overnight dialysis,
sterilizing the mixture by filtration using a 0.22 um sterile syringe filter (Millipore), and transferring the mixture into a sterile and enzyme-free centrifuge tube. The mRNA loading amount and encapsulation rate of the filtered mRNA-LNP are detected using a Ribogreen detection reagent (thermo). The zeta average particle size of the filtered mRNA-LNP is detected using a PCS detector.

In some aspects, the drug may be prepared by:
weighing 1.5-1.8 mg of phospholipid, 1.7-1.9 mg of cholesterol, and 0.8-1 mg of polyethylene glycol lipid, and dissolving them in 0.8-1.5 mL of anhydrous ethanol at 55-65°C to obtain a stock solution;
adding 13-15 mg of a cationic lipid compound to the stock solution at room temperature, and mixing the mixture, to obtain an alcoholic lipid phase;
diluting the nucleic acid drug in an acidic buffer saline solution to a final concentration of 0.5-0.7 mg/mL, fully mixing the mixture to obtain an aqueous phase of nucleic acid;
mixing the alcoholic lipid phase and the aqueous phase of nucleic acid at a volume ratio of 1:(2-5) at room temperature in a mixer to obtain a drug intermediate, dialyzing the drug intermediate through a dialysis apparatus to remove the solvent, and replacing it with a Tris solution containing 0.2%-0.5% sodium chloride and 4%-6% sucrose at a concentration of 18-22 mM, to obtain the drug.

In some aspects, the drug may be prepared by:
weighing 1.5-1.8 mg of phospholipid, 1.7-1.9 mg of cholesterol, and 0.8-1 mg of polyethylene glycol lipid, and dissolving them in 0.8-1.5 mL of anhydrous ethanol at 55-65°C to obtain a stock solution;
adding 13-15 mg of a cationic lipid compound to the stock solution at room temperature, and mixing the mixture, to obtain an alcoholic lipid phase;
diluting the nucleic acid drug in an acidic buffer saline solution to a final concentration of 0.05-0.3 mg/mL, fully mixing the mixture to obtain an aqueous phase of nucleic acid;
mixing the alcoholic lipid phase and the aqueous phase of nucleic acid at a volume ratio of 1:(2-5) at room temperature in a mixer to obtain a drug intermediate, dialyzing the drug intermediate through a dialysis apparatus to remove the solvent, and replacing it with a Tris solution containing 0.2%-0.5% sodium chloride and 4%-6% sucrose at a concentration of 18-22 mM, to obtain the drug.

### Listed Examples

The examples listed below represent some aspects of the present disclosure
Example 1. A cationic lipid compound, comprising a compound represented by formula I; 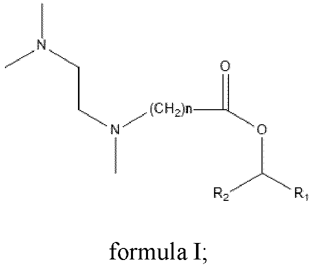
   wherein, in the formula I, n=1, 2, 3, or 4; and
   R₁ and R₂ are each independently selected from a secondary long-chain alkyl ester represented by formula II; 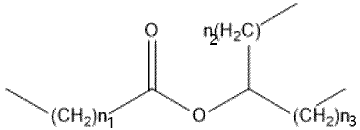 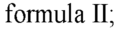
   wherein, in the formula II, n₁=2, 4, or 6; n₂=5, 7, 9, or 11; and n₃=5, 7, 9, or 11.
Example 2. The cationic lipid compound according to Example 1, wherein the cationic lipid compound comprises a compound represented by formula III, formula IV, formula V, formula VI, or formula VII; 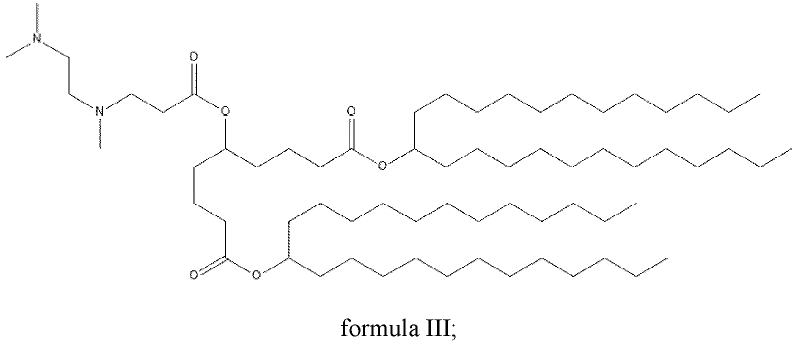 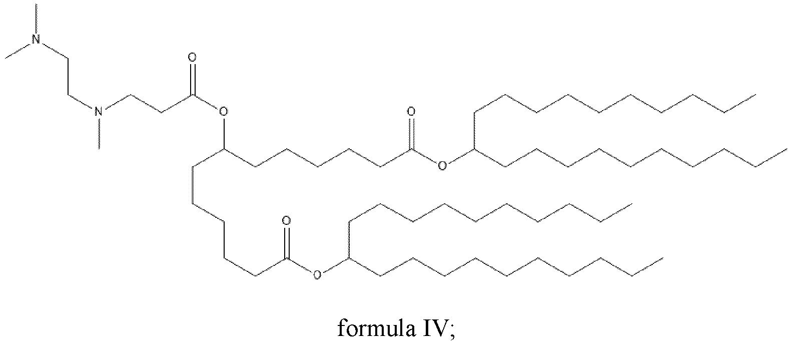 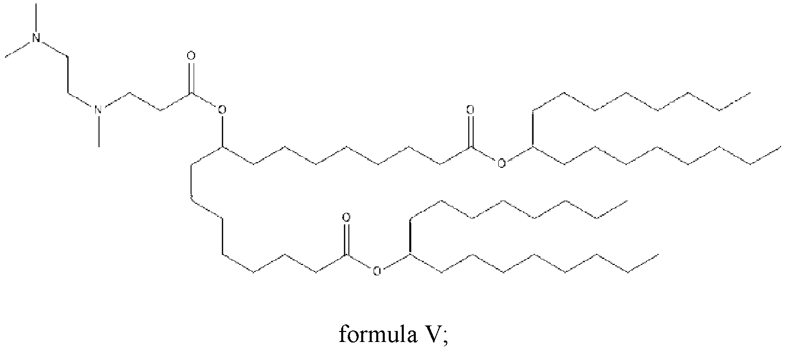 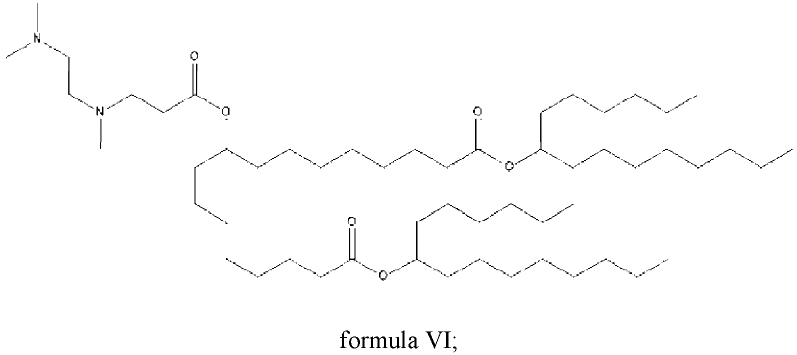 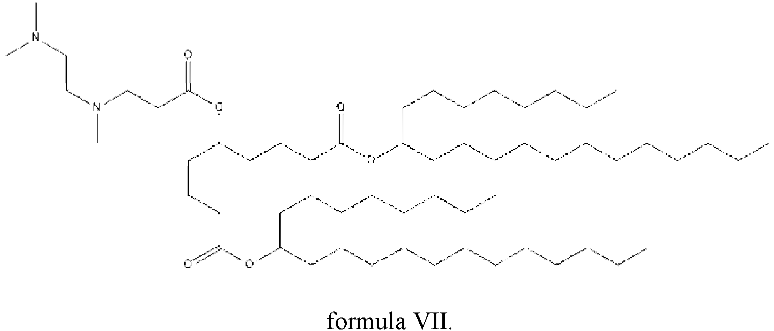
Example 3. A method for preparing the cationic lipid compound according to Example 1 or 2, comprising steps of:
   sequentially performing an alkylation reaction and a reduction reaction with a bromoester compound as a raw material,
   followed by a condensation reaction with an acyl chloride compound, sequentially performing a two-step substitution reaction and a hydrolysis reaction, and finally performing an esterification reaction with a monohydric secondary alcohol compound to obtain the cationic lipid compound.
Example 4. A LNP carrier, comprising any one or a combination of at least two of the cationic lipid compound according to Example 1 or 2.
Example 5. The LNP carrier according to Example 4, wherein the LNP carrier comprises 15%-70% of the cationic lipid compound by molar fraction; and
   preferably, the LNP carrier further comprises 8%-30% of phospholipid, 5%-65% of cholesterol, and 1.5%-3% of polyethylene glycol lipid by molar fraction.
Example 6. A drug, comprising the LNP carrier according to Example 4 or 5.
Example 7. The drug according to Example 6, wherein the drug further comprises a nucleic acid drug; and
   preferably, in the drug, a molar ratio of nitrogen content of the cationic lipid compound in a LNP carrier to phosphorus content in the nucleic acid drug is (2-6): 1.
Example 8. A method for preparing the drug according to Example 6 or 7, comprising steps of:
   weighing and dissolving ingredients other than the cationic lipid compound, to obtain a stock solution;
   adding the cationic lipid compound to the stock solution, and fully mixing the mixture to obtain an alcoholic lipid phase;
   diluting a nucleic acid drug to obtain an aqueous phase of nucleic acid;
   mixing the alcoholic lipid phase and the aqueous phase of nucleic acid to obtain a drug intermediate, and dialyzing the drug intermediate to obtain the drug.
Example 9. A vaccine, comprising the LNP carrier according to Example 4 or 5.
Example 10. Use of the cationic lipid compound according to Example 1 or 2 in the preparation of a LNP carrier, a drug, or a vaccine, or use of the LNP carrier according to Example 4 or 5 in the preparation of a drug and/or a vaccine.

The present disclosure will be further described in detail below with reference to FIGS. 1-9, Preparation Examples 1-2, and Examples 1-2.

### Preparation Examples

### Preparation Example 1

This preparation example provides a cationic lipid compound TM3. The cationic lipid compound TM3 has a structural formula as shown in formula V.

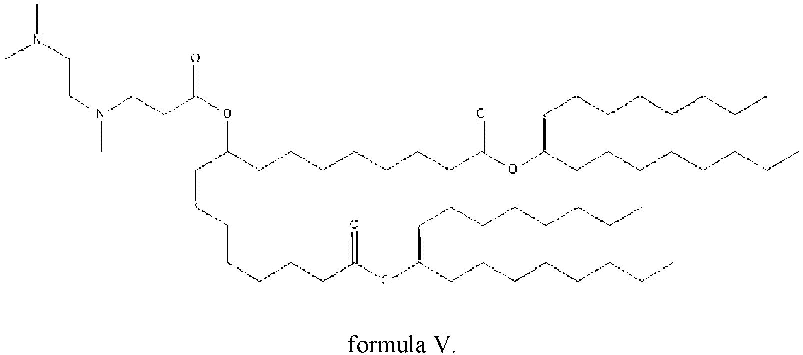

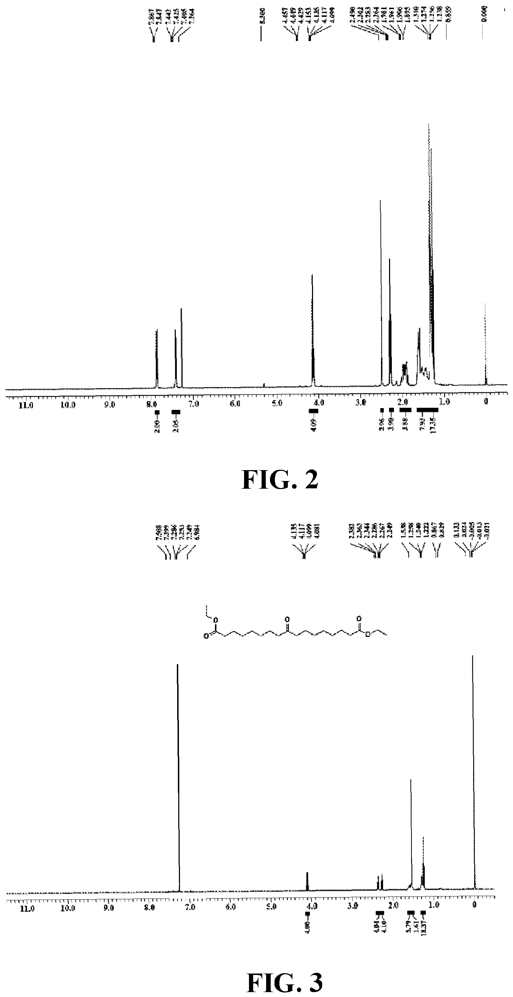

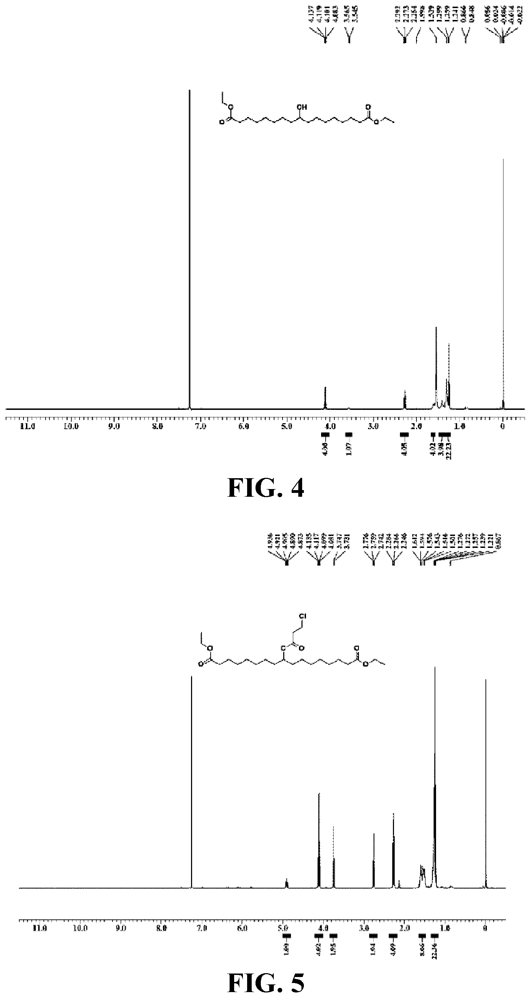

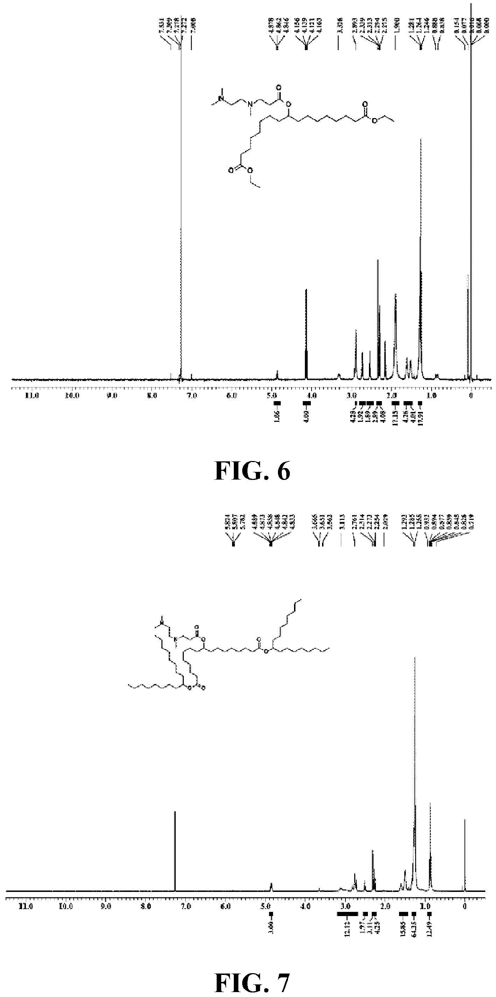

The cationic lipid compound TM3 is prepared in accordance with the following method, following a synthetic route as shown in FIG. 1:
(1) generating an intermediate product 2 through an alkylation reaction of ethyl 8-bromooctanoate with TosMIC and NaH in a DMSO solution with TBAI as a catalyst at room temperature:
   In a 500 mL single-necked flask, 5 g of ethyl 8-bromooctanoate (20 mmol) was weighed and dissolved in 60 mL of anhydrous DMSO. The mixture was stirred at 10°C for 7 min, 1.9 g of TosMIC (10 mmol) was added, the mixture was stirred for 7 min, 1 g of NaH (25 mmol) was added batchwise, and finally 0.7 g of TBAI (2 mmol) was added. The mixture was slowly warmed from 10°C to room temperature, and stirred for 2 h.
   The reaction extent was monitored by TLC. When the reaction was complete, the system was cooled in an ice water bath, and 150 mL of ice water was added to quench the reaction. Then, the mixture was extracted 3 times with 100 mL of DCM each time. All organic phases were collected, washed with 100 mL of water, then washed twice with 150 mL of saturated sodium bicarbonate each time, dried, concentrated, and then purified through a silica gel column to provide the intermediate product 2. A hydrogen spectrum detection result of the intermediate product 2 is as shown in FIG. 2.
(2) generating an intermediate product 3 through a reaction of the intermediate product 2 in an acidic mixed solution of DCM and concentrated sulfuric acid (volume ratio: 5:1) at room temperature:
   In a 250 mL single-necked flask, 4.1 g of the intermediate product 2 was weighed, and dissolved in 50 mL of a DCM solution. The mixture was stirred for 5 min. 10 mL of concentrated sulfuric acid was added, and the mixture was stirred at room temperature for 3 h.
   The reaction extent was monitored by TLC. After the reaction was complete, 50 mL of water was added to the system. The mixture was fully mixed, and then left to stand for layering. The aqueous layer was extracted with 50 mL of DCM. All organic phases were combined, eluted with 50 mL of saturated sodium bicarbonate, dried, concentrated, and then purified through a silica gel column to provide the intermediate product 3. A hydrogen spectrum detection result of the intermediate product 3 is as shown in FIG. 3.
(3) generating an intermediate product 4 through a reduction reaction of the intermediate product 3 with NaBH₄ in a mixed solution of THF and ethanol (volume ratio: 3:1) at room temperature:
   In a 250 mL single-necked flask, 1.5 g of the intermediate product 3 (4 mmol) was weighed, and dissolved in 50 mL of a mixed solution of THF and ethanol (volume ratio: 3:1). The mixture was stirred at 0 °C for 5 min. 0.15 g of NaBH₄ (4 mmol) was slowly added batchwise, and the mixture was kept at room temperature for reaction for 4 h.
   The reaction extent was monitored by TLC. After the reaction was complete, 100 mL of ice water was added to the system to quench the reaction. The mixture was extracted 3 times with 100 mL of DCM each time. All organic phases were combined, eluted with 50 mL of saturated sodium bicarbonate, dried, concentrated, and then purified through a silica gel column to provide the intermediate product 4. A hydrogen spectrum detection result of the intermediate product 4 is as shown in FIG. 4.
(4) generating an intermediate product 5 through a condensation reaction of the intermediate product 4 with 3-chloropropionyl chloride in a pyridine-containing DCM solution (pyridine concentration: 100 mM) at room temperature:
   In a 250 mL single-necked flask, 1.8 g of the intermediate product 4 (5 mmol) was weighed, and dissolved in 50 mL of a DCM solution. Then, 0.4 g of pyridine (5 mmol) was added, the mixture was stirred at 0°C for 5 min, 1 g of 3-chloropropionyl chloride (8 mmol) was slowly added, and the mixture was kept at room temperature for reaction for 1 h.
   The reaction extent was monitored by TLC. After the reaction was complete, 100 mL of ice water was added to the system to quench the reaction. The mixture was washed with 100 mL of water, then washed twice with 150 mL of a saturated sodium bicarbonate solution each time, dried, concentrated, and then purified through a silica gel column to provide the intermediate product 5. A hydrogen spectrum detection result of the intermediate product 5 is as shown in FIG. 5.
(5) generating an intermediate product 6 through a substitution reaction of the intermediate product 5 with NaI in an acetone solution at 70°C:
   In a 250 mL single-necked flask, 1.8 g of the intermediate product 5 (4 mmol) was weighed and dissolved in 50 mL of an acetone solution. 0.8 g of NaI (5 mmol) was slowly added. The mixture was heated to a temperature of 70°C for reaction for 16 h.
   The reaction extent was monitored by TLC. After the reaction was complete, the organic phase was eluted with 50 mL of saturated sodium thiosulfate, dried, concentrated, and then purified through a silica gel column to provide the intermediate product 6.
(6) generating an intermediate product 7 through a substitution reaction of the intermediate product 6 with N,N,N'-trimethylethylenediamine in a DCM solution at room temperature:
   In a 250 mL single-necked flask, 2.2 g of the intermediate product 6 (4 mmol) was weighed and dissolved in 50 mL of a DCM solution. 0.6 g of N,N,N'-trimethylethylenediamine (6 mmol) was added. The mixture was stirred at room temperature for reaction for 2 h.
   The reaction extent was monitored by TLC. After the reaction was complete, the organic phase was eluted with 50 mL of saturated sodium bicarbonate, dried, concentrated, and then purified through a silica gel column to provide the intermediate product 7. A hydrogen spectrum detection result of the intermediate product 7 is as shown in FIG. 6.
(7) generating an intermediate product 8 through a hydrolysis reaction of the intermediate product 7 with LiOH in a mixed solution of ethanol and water (volume ratio: 1:2) at room temperature:
   In a 250 mL single-necked flask, 3.8 g of the intermediate product 7 (5 mmol) and 0.5 g of LiOH (20 mmoL) were weighed, and dissolved in 100 mL of a mixed solution of ethanol and water (volume ratio 1:2). The mixture was stirred at room temperature for 5 h.
   The reaction extent was monitored by TLC. After the reaction was complete, and after ethanol was spin-dried, the mixture was acidized with 2 M of hydrochloric acid to a pH of about 2, and extracted 3 times with 100 mL of DCM each time. All organic phases were combined, eluted with 50 mL of saturated sodium bicarbonate, dried, concentrated, and then purified through a silica gel column to provide the intermediate product 8.
(8) generating the cationic lipid compound TM3 through an esterification reaction of the intermediate product 8 with 9-heptadecanol in a DCM solution under the action of DMAP and DCC at room temperature:
   In a 250 mL single-necked flask, 3.6 g of the intermediate product 8 (5 mmol), 1.8 g of DMAP (15 mmol), and 4.6 g of 9-heptadecanol (18 mmol) were weighed, and dissolved in 60 mL of DCM. The mixture was stirred for 5 min, and 3.1 g of DCC (15 mmol) was added. The mixture was stirred at room temperature overnight.
   The reaction extent was monitored by TLC. After the reaction was complete, the mixture was washed 3 times with 60 mL of water each time, then washed twice with 50 mL of saturated sodium bicarbonate each time, dried, concentrated, and then purified through a silica gel column to provide the cationic lipid compound TM3. A hydrogen spectrum detection result of the TM3 is as shown in FIG. 7.

In the above reactions, statistical results of the mass and yield of the product obtained in each step are as shown in Table 1.

**Table 1 Statistical results of mass and yield of product obtained in each step**

| Product | Mass (g) | Yield (%) |
|---|---|---|
| Intermediate product 2 | 4.7 | 94 |
| Intermediate product 3 | 3.7 | 62 |
| Intermediate product 4 | 1.0 | 67 |
| Intermediate product 5 | 1.9 | 82 |
| Intermediate product 6 | 1.8 | 81 |
| Intermediate product 7 | 1.8 | 60 |
| Intermediate product 8 | 3 | 83 |
| TM3 | 3.3 | 70 |

### Preparation Example 2

This preparation example provides a drug TP1, wherein a nucleic acid drug is a mRNA encoding SARS-CoV-2 spike glycoprotein (S protein), and is prepared in accordance with the following method:
1.64 mg of HSPC, 1.83 mg of cholesterol, and 0.89 mg of DSPE-PEG2000 were weighed, and dissolved in 1 mL of anhydrous ethanol at 60°C to obtain a stock solution.
13.41 mg of TM3 was added to the stock solution at room temperature, and the mixture was mixed to obtain an alcoholic lipid phase.
1.6 mL of 1 mg/mL mRNA was dissolved in 1.4 mL of a D-citric acid solution at a pH of 4.0, and the mixture was fully mixed, to obtain an aqueous phase of nucleic acid.
1 mL of the alcoholic lipid phase and 3 mL of the aqueous phase of nucleic acid were mixed in a mixer at room temperature to obtain a drug intermediate.

The intermediate was dialyzed through a dialysis apparatus to remove ethanol and citric acid, and was replaced with a Tris solution containing 0.4% sodium chloride and 5% sucrose at a concentration of 20 mM to provide the drug TP1.

### Performance test

The drug TP1 prepared in Preparation Example 2 was subjected to performance test, including particle size detection, polydispersity detection, and encapsulation rate detection.

### Particle size and polydispersity detection

The particle size and polydispersity (PDI) of nanoparticles were determined using a laser scattering particle size analyzer (PCS, Dandong BeNano 180 Zeta pro laser particle size analyzer) with incident light from a 671 nm solid laser. The kinetic light scattering test was performed at 25°C with 173° backscattering detection using a scattered light path. An average value of 3 consecutive tests was used as the test data.

### Encapsulation rate detection

Encapsulation rate was detected by fluorescent quantitative detection using Quant-it^{™} RiboGreen RNA Assay Kit as the assay kit. Triton was added for demulsification and then releasing nucleic acid in the formulation. A specific nucleic acid dye ribogreen was added. ALLSHENG Feyond-A300 microplate reader with the excitation light set to a wavelength of 470 nm and the emission light set to a wavelength of 525 nm was used to detect the absorbance values of samples and calculate the total content of nucleic acid based on a standard curve. Further, the specific nucleic acid dye ribogreen was added to LNP samples treated without addition of Triton, and the ALLSHENG Feyond-A300 microplate reader with the excitation light set to a wavelength of 470 nm and the emission light set to a wavelength of 525 nm was used to detect the absorbance values of the samples, and calculate the content of free nucleic acid based on the standard curve as per a formula as follows: Encapsulation rate=(total content of nucleic acid-content of free nucleic acid)/total content of nucleic acid×100%.

Detection results of particle size, polydispersity, and encapsulation rate are as shown in Table 2.

**Table 2 Detection results of particle size, polydispersity, and encapsulation rate of TP1**

| Drug | N/P | TM3 | HSPC | Cholesterol | DSPE-PEG2000 | Particle size | PDI | Encapsulation rate |
|---|---|---|---|---|---|---|---|---|
| TP1 | 6 | 66.4% | 9.8% | 22.3% | 1.5% | 169 nm | 0.07 | 88% |

As can be seen from Table 2, the drug TP1 prepared according to the recipe and method of Preparation Example 2 has an average particle size of 169 nm, and a PDI of 0.07, has an appropriate particle size and uniform particle size distribution, and also has a high encapsulation rate, indicating that it has good drug loading capacity, thereby creating conditions for subsequent practical use.

### Preparation Example 3

This preparation example provides the preparation of seven drugs, wherein the nucleic acid drug is a mRNA encoding luciferase. Specific lipid recipe information is as shown in Table 3.

**Table 3. Lipid recipe information for seven TM3-containing LNP drugs**

| | HG-06 | HG-10 | HG-21 | HG-23 | HG-25 | HG-28 | HG-30 |
|---|---|---|---|---|---|---|---|
| TM3 | 65.5% | 64% | 65% | 29.7% | 29.67% | 40.52% | 40.52% |
| HSPC | 13% | 6.7% | 13.05% | 10.47% | 10.59% | 14.28% | 14.28% |
| Cholesterol | 20% | 27% | 19.65% | 58.32% | 58.27% | 44.51% | 44.51% |
| DMG-PEG | 1.5% | 1.5% | 2.31% | 1.51% | 1.47% | 0.69% | 0.69% |

Preparation method: accurately weighing a lipid based on the above recipe, and dissolving the lipid in anhydrous ethanol while stirring on a magnetic stirrer, adding a calculated amount of a citrate buffer at pH=4 to a mRNA stock solution, fully mixing the mixture, sucking the above ethanolic lipid solution and an aqueous solution of mRNA-citrate buffer with a syringe, starting a LNP mixing pump to collect a solution in a stable mixing stage, adding the above mixed mRNA-LNP intermediate into a dialysis bag, adding a dialysis buffer pre-cooled at 4°C, transferring the dialysis bag into a 2-8°C refrigerator, stirring at 200 rpm for overnight dialysis, sterilizing the mixture by filtration using a 0.22 um sterile syringe filter (Millipore), transferring the mixture into a sterile and enzyme-free centrifuge tube.

The mRNA loading amount and encapsulation rate of the filtered mRNA-LNP were detected using a Ribogreen detection reagent (thermo). The zeta average particle size of the filtered mRNA-LNP was detected using a PCS detector. The test result is as shown in Table 4.

**Table 4 Detection results of particle size and encapsulation rate of seven TM3-containing drugs**

| | HG5-06 | HG5-10 | HG5-21 | HG5-23 | HG5-25 | HG5-28 | HG5-30 |
|---|---|---|---|---|---|---|---|
| Drug loading amount (wt.) | 5.75% | 4.64% | 4.52% | 2.81% | 4.52% | 5.65% | 1.32% |
| Encapsulation rate | 98.6% | 96.3% | >97.0% | 99.0% | 98.6% | 99.0% | 98.5% |
| Average particle size | 82.23 | 106.25 | 110.12 | 59.32 | 80.62 | 122.84 | 145.86 |
| PDI | 0.046 | 0.056 | 0.039 | 0.076 | 0.006 | 0.039 | 0.042 |

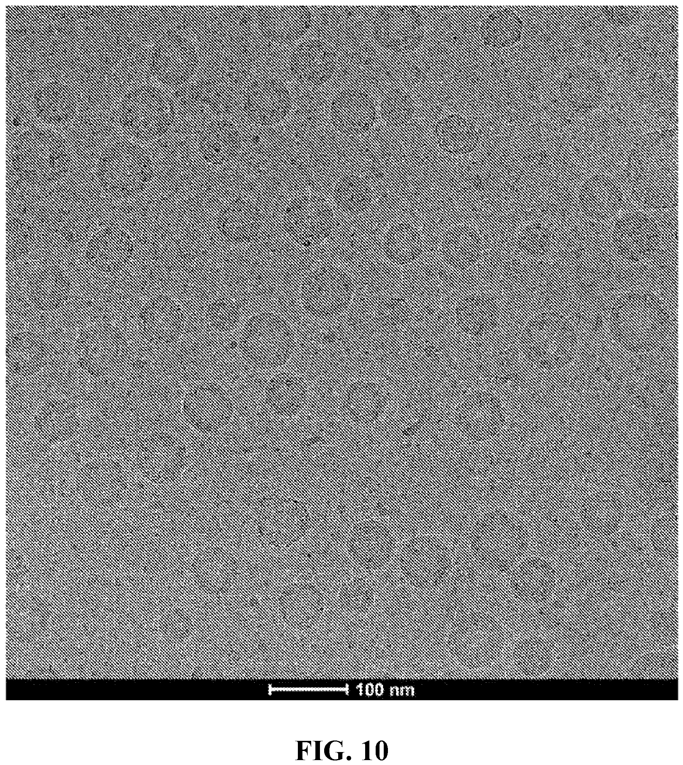

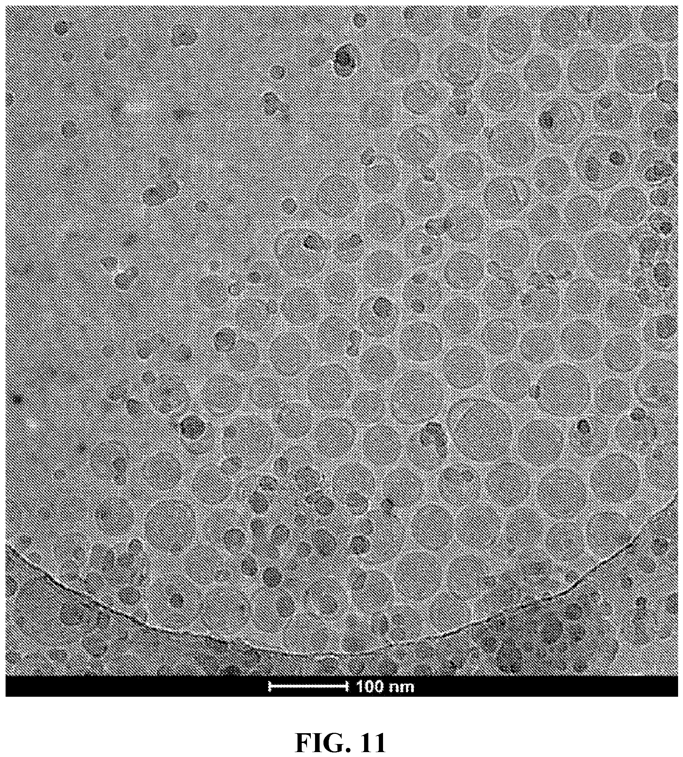

Cryo-electron microscope: (2.5 µL of a LNP solution was dropped onto a grid (Quantifoil Cu R1.2/1.3, 300 mesh), to prepare cryo-electron microscope samples using Vitrobot Mark IV (ThermoFisher Scientific), which were imaged with Talos F200C equipped with a Ceta 4k × 4k camera. The cryo-electron microscope picture of the drug HG5-06 is as shown in FIG. 10. The cryo-electron microscope picture of the drug HG5-023 is as shown in FIG. 11.

### Preparation Example 4

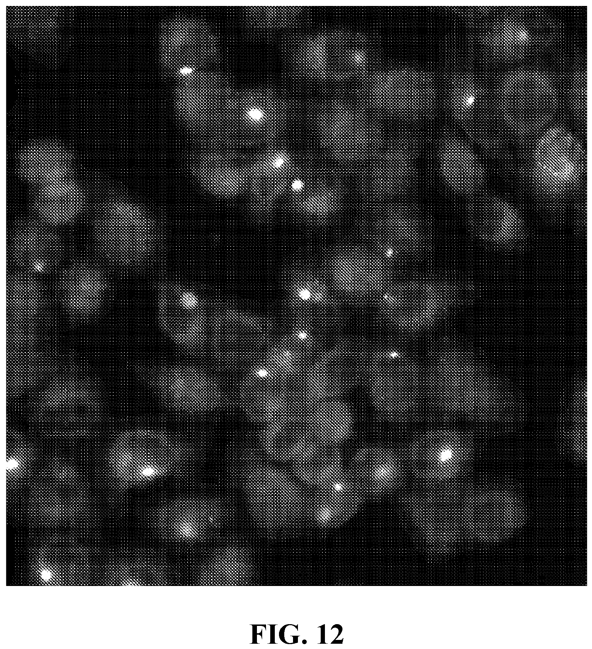

Using a method similar to Preparation Example 3, this preparation example provides the preparation of a TM6-containing drug and a TM7-containing drug, wherein the nucleic acid drug is a mRNA encoding SARS-CoV-2 spike glycoprotein (S protein). The NMR spectrum of TM7 is as shown in FIG. 12.

The recipe information, particle size, and encapsulation rate information of the resulting LNP are as shown in Table 5.

**Table 5 Detection results of TM6-containing drug and TM7-containing drug and particle size thereof**

| | Cationic lipid | HSPC | Chol | PEG-DMG | Encapsulation rate | Particle size | PDI |
|---|---|---|---|---|---|---|---|
| HG5-06-TM6 | 50% | 10% | 38.5% | 1.5% | 95.26 | 117.94 | 0.085 |
| HG5-21-TM6 | 65% | 13.05% | 19.65% | 2.31% | 85.46 | 164.12 | 0.041 |
| HG5-23-TM6 | 29.7% | 10.47% | 58.32% | 1.51% | 98.44 | 88.21 | 0.097 |
| HG5-06-TM7 | 50% | 10% | 38.5% | 1.5% | 96.95 | 107.11 | 0.065 |
| HG5-21-TM7 | 65% | 13.05% | 19.65% | 2.31% | 77.57 | 202.03 | 0.121 |
| HG5-23-TM7 | 29.7% | 10.47% | 58.32% | 1.51% | 98.9 | 76.28 | 0.055 |

### Example 1

In this example, an expression amount of the drug TP1 prepared in Preparation Example 2 in cells cultured in vitro was detected, including the following steps:

### (1) Cell culture and collection:

As a derivative of a human embryonic kidney cell strain 293, a cell line 293T was obtained by passage in a laboratory, was cultured in RPMI-1640 medium containing 10% FBS, and was passaged 2-3 times every week. Cells with a cell density of about 80% were spread on a plate, counted, and adjusted with a culture medium to a cell concentration of 2×10⁵/mL. In a 48-well plate, 0.5 mL of the above cell solution was added to each well, i.e., the cell count in each well was approximately 1×10⁵. 3 duplicate wells were set for each group, and 3 duplicate wells were also set for the blank control group.

### (2) Co-incubation of cells and drugs:

The total concentration of mRNA was detected based on the fluorescence of the mRNA. The drug containing 1 µg of mRNA was added to each well, and incubated for 18, 24, 48, and 72 h respectively, to observe the protein expression amounts at different incubation time.

### (3) Protein expression detection:

The cell culture plate was centrifuged at 800 g for 5 min, the supernatant was diluted 10 times, 50 times, and 100 times respectively with ddH₂O to prepare to-be-tested samples. According to the expressed protein, a standard curve was plotted with SARS-CoV-2 spike glycoprotein (S protein) as the standard substance, and detection was performed by ELISA.

### (4) Result analysis:

The OD value of the blank control was subtracted from the OD values of the to-be-tested samples to calibrate absorbance values of the standard curve. The standard curve was plotted with standard concentrations as the x-axis, to calculate S protein concentrations corresponding to the OD values of the samples based on the standard curve.

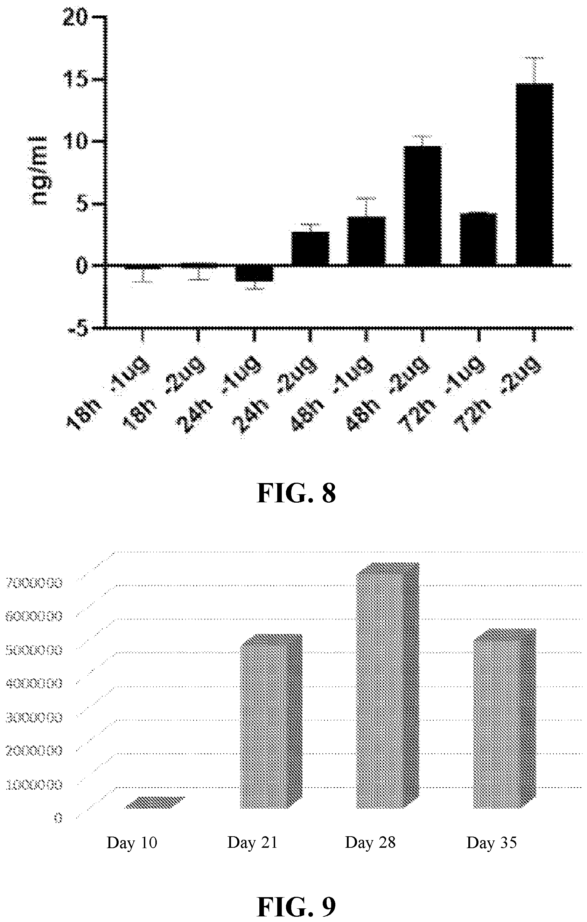

A detection result of a protein expression amount of the drug TP1 in 293T cells is as shown in FIG. 8. As can be seen from FIG. 8, the drug TP1 can be effectively transferred into 293T cells to express the S protein. In addition, the expression amount continues to increase over the incubation time, and the protein expression amount is also positively correlated with the dose. The above result shows that the LNP carrier can transfer the drug into cells and release the drug, so that it can be used in the treatment of an associated disease, and has practical use value.

### Example 2

In this example, in vivo immunogenicity of the drug TP1 prepared in Preparation Example 2 was detected, including the following steps:
6 female BALB/c mice aged 6-8 weeks were intramuscularly (i.m.) injected with the drug TP1 on days 1 and 14 respectively. An anti-S protein antibody titer in mouse serum was detected by ELISA on days 10, 21, 28, and 35 after first dosing.

A detection result of the anti-S protein antibody titer in mouse serum is as shown in FIG. 9. As can be seen from FIG. 9, antibodies were produced on day 10 after the first dosing. After booster injection was given on day 14, the antibody expression amount was increased significantly on day 21. The antibodies continued to be expressed over time, and were still expressed on day 35. The above result shows that the drug can effectively activate immune response of the animals, and the antibody titer in the animals was significantly increased after dosing. Therefore, it can be used as a vaccine to stimulate the organisms to produce corresponding immune response, and achieves significant effects.

### Example 3

In this example, the efficacy of TM6 drug and TM7 drug in different recipes in cells and animal models in Preparation Example 4 was investigated.

Evaluation of the in vitro cell transfection activity of the formulation: Frozen cells were defrozen at 37°C, transferred to a 5 mL culture solution, and centrifuged at 1,000 rpm for 5 min. Then, the supernatant was discarded, and the cells were resuspended in the culture solution. 10 µL of the culture solution was stained with trypan blue, to obtain the count/viability rate. The remaining cell suspension was transferred to a cell culture flask, which was placed in a 37°C incubator for static culture. When the cells grew to about 90% abundance, the cells were collected by digestion and centrifugation, resuspended, then counted, and adjusted to a concentration of 2*10^5/ml. 0.5 ml of the cell suspension was added to each well of a 48-well plate (corning). Groups were set up according to specific experiments, and 3-4 duplicate wells were set for each group. mRNA-LNP corresponding to 1 ug of mRNA was added to each well. After dosing, the cell culture plate was shaken by blowing, and transferred back to a cell incubator to continue culturing. After cell culture for 24 h, the cell culture supernatant was transferred to a corresponding EP tube, and centrifuged at 1300 rpm for 5 min for detection.

Detection of reporter gene concentration: Samples collected from the above cell culture were diluted at a certain ratio, and the dilution ratio was determined through a preliminary experiment. The diluted samples were tested using an Elisa kit.

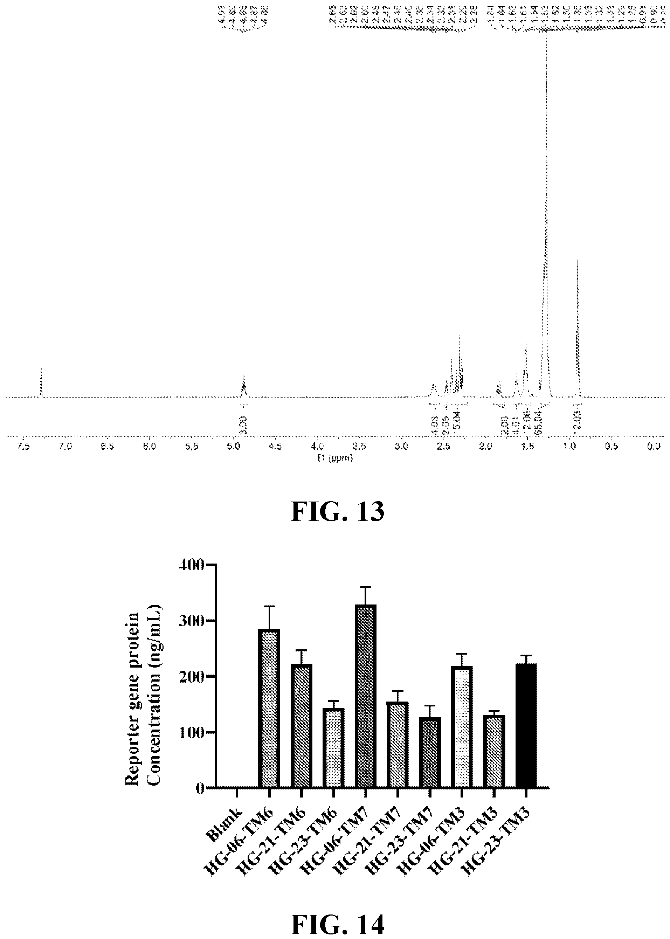

FIG. 14 shows that the TM6-containing drug and TM7-containing drug have slightly higher transfection activity thanTM3-containing drug based on comparison of cell transfection effects of TM6 and TM7 drugs in a plurality of lipid recipes and control with drugs in similar TM3-containing lipid recipes. However, the TM3 drug in HG-23 lipid recipe also has very high activity. To sum up, although the particle size of LNPs composed of cations of TM series is different to a certain extent at different lipid ratios, they all show better cell transfection activity.

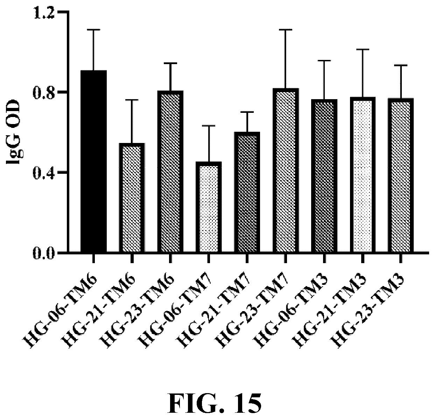

Immune effects of these drugs in mice were further evaluated. The animal models were female Balb/c mice aged 6-8 weeks and bred in an SPF-level animal breeding center. According to the trial protocol, the animals were administered i.m., with n=6 in each group, and with a dosage of 10 ug per animal. Two weeks after immunization, orbital blood was collected after anesthesia. About 50 ul of whole blood was collected in a 1.5 mL EP tube, placed at room temperature for 0.5 h, and then centrifuged at 7,000 g for 10 min. After collection and dilution at a certain ratio, the OD value was detected using (SARS-Cov2-2 Spike RBD Antibody Titer Assay Kit (mouse) SinoBiological). The result is as shown in FIG. 15.

According to the analysis of the result of the mouse experiment, three lipids with different structures and their recipes induced equivalent titers of antibodies against COVID S protein, wherein the HG-06 recipe of TM6 and the HG-23 recipe of TM7 both had excellent performance, and had clinical development value.

**Example 4:** TMEA is a cationic lipid structure disclosed in CN 105085292 B, has same hydrophilic and ionizable end groups as TM3 (both are 3-((2-(dimethylamino)ethyl)(methyl)amino)propionic acid), but has a different hydrophobic structure. Therefore, a mRNA-loaded LNP constructed therewith was compared. As can be seen from the comparison of three different lipid recipes in Table 6, the LNP obtained from TM3 has much better in vivo protein expression activity than the TMEA LNP.

Balb/c mice aged 6-8 weeks were selected for the in vivo protein expression experiments. Each mouse was intravenously administered with 10 ug of nucleic acid-loaded LNP drug. Blood samples were collected 6 h after administration, and the protein content in the serum was detected using an Elisa kit.

**Table 6. Comparative study of TMEA and lipid TM3**

| | TMEAa | TMEAb | TMEAc | TM3a | TM3b | TM3c |
|---|---|---|---|---|---|---|
| TMEA | 50.0% | 40.0% | 65.5% | / | / | / |
| TM3 | / | / | / | 50.0% | 40.0% | 65.5% |
| DSPC | 10.0% | 18.5% | 13.0% | 10.0% | 18.5% | 13.0% |
| CHOL | 38.5% | 40.0% | 20.0% | 38.5% | 40.0% | 20.0% |
| DMG-PEG2000 | 1.5% | 1.5% | 1.5% | 1.5% | 1.5% | 1.5% |
| Particle size (nm) | 107.45 | 99.23 | 89.54 | 81.79 | 70.97 | 62.11 |
| PDI | 0.096 | 0.145 | 0.191 | 0.054 | 0.104 | 0.088 |
| Encapsulation rate | 95.16% | 94.08% | 97.50% | 96.94% | 96.89% | 98.91 |
| In vivo protein expression (ng/ml) | 942±488 | 753±195 | 229±43 | 7861±3004 | 2454±691 | 1211±622 |

This specific embodiment is only an explanation of the present disclosure, and does not impose any limitation on the present disclosure. After reading this specification, those skilled in the art can make modifications to this embodiment without creative contributions as needed, but the modifications, as long as being encompassed within the scope of claims of the present disclosure, are all protected by the Patent Law.

## Claims

1. A cationic lipid compound, comprising a compound represented by formula I; 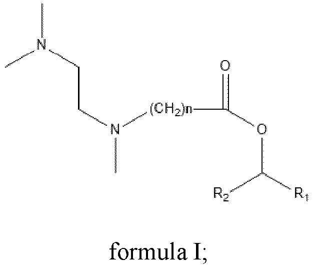
wherein, in the formula I, n=1, 2, 3, or 4; and
R₁ and R₂ are each independently selected from a secondary long-chain alkyl ester represented by formula II; 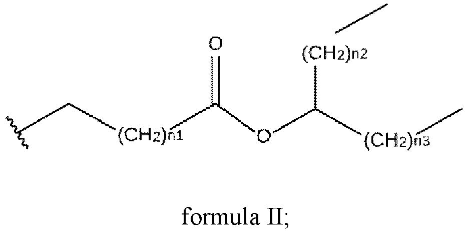
wherein, in the formula II, n₁=2, 4, or 6; n₂=5, 7, 9, or 11; n₃=5, 7, 9, or 11; and a wavy line represents a connection point between the formula II and other moieties of the formula I.

2. The cationic lipid compound according to any one of claim 1, wherein n=1, 2, or 3.

3. The cationic lipid compound according to any one of claim 1 or 2, wherein n=2.

4. The cationic lipid compound according to any one of claims 1-3, wherein the n1 in the R₁ has a same value as the n1 in the R₂.

5. The cationic lipid compound according to claim 1, wherein the cationic lipid compound comprises a compound represented by formula III, formula IV, formula V, formula VI, formula VII, formula VIII, or formula IX;

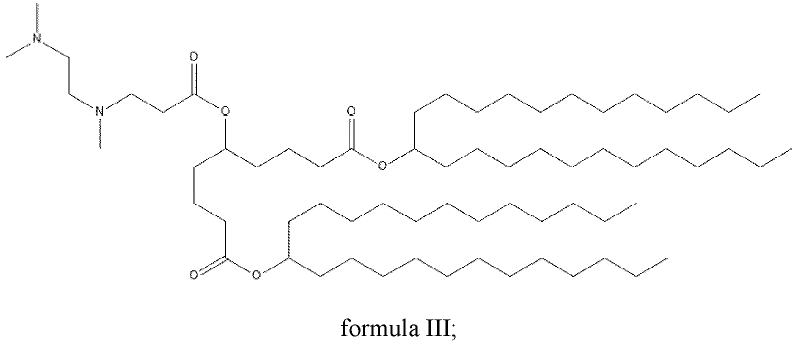

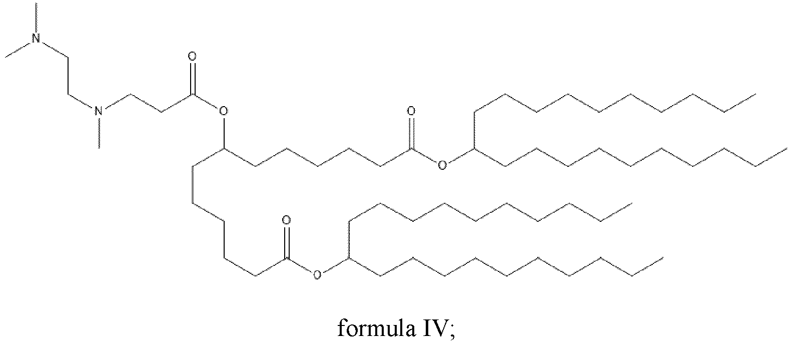

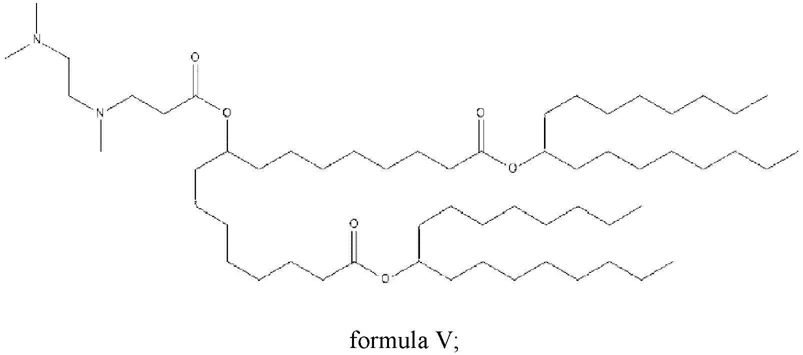

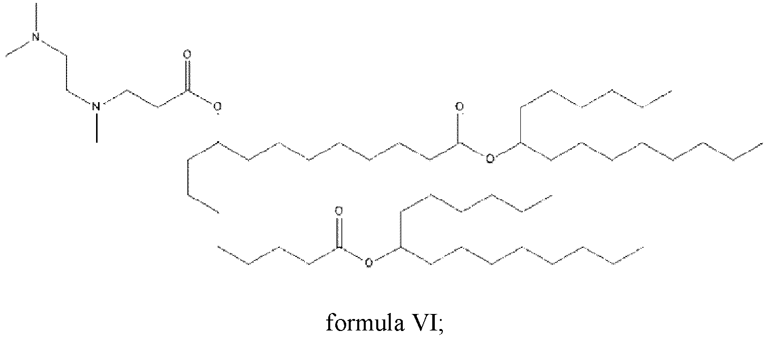

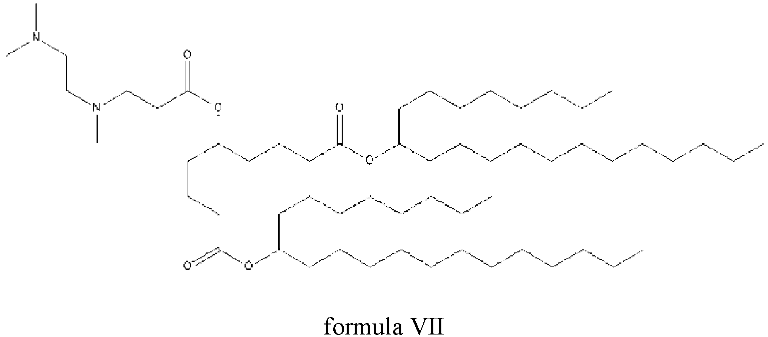

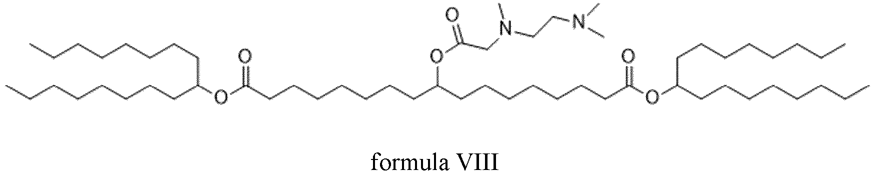

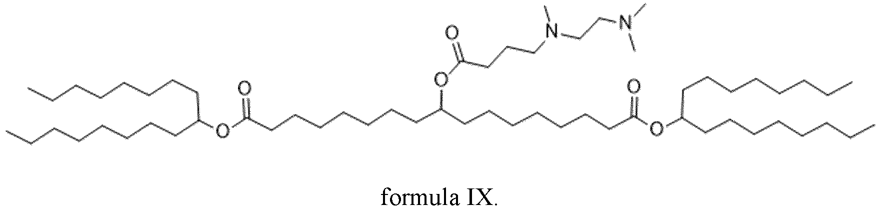

6. A method for preparing the cationic lipid compound according to any one of claims 1-5, comprising steps of:
sequentially performing an alkylation reaction and a reduction reaction with a bromoester compound as a raw material,
followed by a condensation reaction with an acyl chloride compound, sequentially performing a two-step substitution reaction and a hydrolysis reaction, and finally performing an esterification reaction with a monohydric secondary alcohol compound to obtain the cationic lipid compound.

7. A LNP carrier, comprising any one or a combination of at least two of the cationic lipid compound according to any one of claims 1-5.

8. The LNP carrier according to claim 7, wherein the LNP carrier comprises 15%-70% of the cationic lipid compound by molar fraction; and
preferably, the LNP carrier further comprises 5%-40% of phospholipid and 10%-65% of cholesterol by molar fraction.

9. A drug, comprising the LNP carrier according to claim 7 or 8.

10. The drug according to claim 9, wherein the drug further comprises a nucleic acid drug; and preferably, in the drug, a molar ratio of nitrogen content of the cationic lipid compound in the LNP carrier to phosphorus content in the nucleic acid drug is from 2:1 to 15:1.

11. The drug according to claim 10, wherein a molar ratio of the nitrogen content of the cationic lipid compound in the LNP carrier to the phosphorus content in the nucleic acid drug is from 4:1 to 6:1.

12. The drug according to claim 9, wherein the drug further comprises a nucleic acid drug; preferably, in the drug, a mass fraction (drug loading amount) of the nucleic acid drug in the drug-containing LNP is between 1.0-15.0%, and particularly preferably, the drug loading amount of the nucleic acid drug is between 3.0-8.0 wt.%.

13. A method for preparing the drug according to any one of claims 9-12, comprising steps of:
weighing and dissolving ingredients other than the cationic lipid compound, to obtain a stock solution;
adding the cationic lipid compound to the stock solution, and fully mixing the mixture to obtain an alcoholic lipid phase;
diluting a nucleic acid drug to obtain an aqueous phase of nucleic acid;
mixing the alcoholic lipid phase and the aqueous phase of nucleic acid to obtain a drug intermediate, and dialyzing the drug intermediate to obtain the drug.

14. A vaccine, comprising the LNP carrier according to claim 7 or 8.

15. Use of the cationic lipid compound according to any one of claims 1-5 in the preparation of a LNP carrier, a drug, or a vaccine, or use of the LNP carrier according to claim 7 or 8 in the preparation of a drug and/or a vaccine.
